# EUROPEAN PATENT APPLICATION

(11) **EP 2 607 373 A1**
(43) Date of publication of application: **26.06.2013**
(21) Application number: 11195626.4
(22) Date of filing: 23.12.2011
(51) Int. Cl.: C07K 5/10, C07K 1/02

(54) **Liquid phase synthesis of self-assembling peptides to be linked to polymers or to other bioactive and/or self-assembling peptides**

(71) Applicant: SOLVAY SA, 1120 Bruxelles (BE)
(72) Inventor: Moussa, Wafa, 06300 Nice (FR); Jeannin, Laurent, 1090 Brussels (BE); Callens, Roland, 8700 Tielt (BE); Bousmanne, Martin, 1040 Brussels (BE)
(74) Representative: Mross, Stefan P.M.

(57) **Abstract**

The present invention relates to a process for preparing a peptide moiety comprising an amino acid sequence (I)

R-Xaa1-Xaa5-Xaa3-Xaa6-R1 (I),

wherein R1 is selected from OH and NH₂
and a peptide moiety comprising an amino acid sequence (II)

H-Xaa1-Xaa2-Xaa3-Xaa4-Xaal-Xaa5-Xaa3-Xaa6-R1 (II),

wherein R1 is selected from OH and NH₂
whereby Xaa1 and Xaa3 are residues of hydrophobic amino acids and are preferably selected from the group consisting of phenylalanine, tryptophan, histidine, tyrosine, isoleucine, alanine, leucine, norleucine and valine ; and Xaa2, Xaa4, Xaa5 and Xaa6 are residues of charged amino acids and are preferably selected from the group consisting of arginine, aspartic acid, glutamic acid and lysine.

The invention further relates to a process for synthesis of a polymer covalently linked with at least one peptide, wherein the peptide comprises said peptide moiety.

SEQUENCE LISTING

<110> Solvay AS

<120> Liquid phase synthesis of self-assembling peptides to be linked to
polymers or to other bioactive and/or self-assembling peptides

<130> S2011-0230

<160> 139

<170> BiSSAP 1.0

<210> 1
<211> 4
<212> PRT
<213> artificial sequences

<220>
<221> SOURCE
<222> 1..4
<223> /mol_type="protein"
      /note="self-assembling peptides"
      /organism="artificial sequences"

<400> 1
Phe Glu Phe Glu
1

<210> 2
<211> 4
<212> PRT
<213> artificial sequences

<220>
<221> SOURCE
<222> 1..4
<223> /mol_type="protein"
      /note="self-assembling peptide"
      /organism="artificial sequences"

<400> 2
Phe Glu Phe Lys
1

<210> 3
<211> 4
<212> PRT
<213> artificial sequences

<220>
<221> SOURCE
<222> 1..4
<223> /mol_type="protein"
      /note="self-assembling peptide"
      /organism="artificial sequences"

<400> 3
Phe Glu Phe Asp
1

<210> 4
<211> 4
<212> PRT
<213> artificial sequences

<220>
<221> SOURCE
<222> 1..4
<223> /mol_type="protein"
      /note="self-assembling peptide"
      /organism="artificial sequences"

<400> 4
Phe Glu Phe Arg
1

<210> 5
<211> 4
<212> PRT
<213> artificial sequences

<220>
<221> SOURCE
<222> 1..4
<223> /mol_type="protein"
      /note="self-assembling peptide"
      /organism="artificial sequences"

<400> 5
Phe Arg Phe Arg
1

<210> 6
<211> 4
<212> PRT
<213> artificial sequences

<220>
<221> SOURCE
<222> 1..4
<223> /mol_type="protein"
      /note="self-assembling peptide"
      /organism="artificial sequences"

<400> 6
Phe Arg Phe Lys
1

<210> 7
<211> 4
<212> PRT
<213> artificial sequences

<220>
<221> SOURCE
<222> 1..4
<223> /mol_type="protein"
      /note="self-assembling peptide"
      /organism="artificial sequences"

<400> 7
Phe Arg Phe Glu
1

<210> 8
<211> 4
<212> PRT
<213> artificial sequences

<220>
<221> SOURCE
<222> 1..4
<223> /mol_type="protein"
      /note="self-assembling peptide"
      /organism="artificial sequences"

<400> 8
Phe Arg Phe Asp
1

<210> 9
<211> 4
<212> PRT
<213> artificial sequences

<220>
<221> SOURCE
<222> 1..4
<223> /mol_type="protein"
      /note="self-assembling peptide"
      /organism="artificial sequences"

<400> 9
Phe Lys Phe Glu
1

<210> 10
<211> 4
<212> PRT
<213> artificial sequences

<220>
<221> SOURCE
<222> 1..4
<223> /mol_type="protein"
      /note="self-assembling peptide"
      /organism="artificial sequences"

<400> 10
Phe Lys Phe Lys
1

<210> 11
<211> 4
<212> PRT
<213> artificial sequences

<220>
<221> SOURCE
<222> 1..4
<223> /mol_type="protein"
      /note="self-assembling peptide"
      /organism="artificial sequences"

<400> 11
Phe Lys Phe Arg
1

<210> 12
<211> 4
<212> PRT
<213> artificial sequences

<220>

<221> SOURCE
<222> 1..4
<223> /mol_type="protein"
      /note="self-assembling peptide"
      /organism="artificial sequences"

<400> 12
Phe Lys Phe Asp
1

<210> 13
<211> 4
<212> PRT
<213> artificial sequences

<220>
<221> SOURCE
<222> 1..4
<223> /mol_type="protein"
      /note="self-assembling peptide"
      /organism="artificial sequences"

<400> 13
Phe Asp Phe Asp
1

<210> 14
<211> 4
<212> PRT
<213> artificial sequences

<220>
<221> SOURCE
<222> 1..4
<223> /mol_type="protein"
      /note="self-assembling peptide"
      /organism="artificial sequences"

<400> 14
Phe Asp Phe Glu
1

<210> 15
<211> 4
<212> PRT
<213> artificial sequences

<220>
<221> SOURCE
<222> 1..4
<223> /mol_type="protein"
      /note="self-assembling peptide"
      /organism="artificial sequences"

<400> 15
Phe Asp Phe Arg
1

<210> 16
<211> 4
<212> PRT
<213> artificial sequences

<220>
<221> SOURCE

<222> 1..4
<223> /mol_type="protein"
      /note="self-assembling peptide"
      /organism="artificial sequences"

<400> 16
Phe Asp Phe Lys
1

<210> 17
<211> 4
<212> PRT
<213> artificial sequences

<220>
<221> SOURCE
<222> 1..4
<223> /mol_type="protein"
      /note="self-assembling peptide"
      /organism="artificial sequences"

<400> 17
Trp Glu Trp Glu
1

<210> 18
<211> 4
<212> PRT
<213> artificial sequences

<220>
<221> SOURCE
<222> 1..4
<223> /mol_type="protein"
      /note="self-assembling peptide"
      /organism="artificial sequences"

<400> 18
Trp Glu Trp Lys
1

<210> 19
<211> 4
<212> PRT
<213> artificial sequences

<220>
<221> SOURCE
<222> 1..4
<223> /mol_type="protein"
      /note="self-assembling peptide"
      /organism="artificial sequences"

<400> 19
Trp Arg Trp Arg
1

<210> 20
<211> 4
<212> PRT
<213> artificial sequences

<220>
<221> SOURCE
<222> 1..4

<223> /mol_type="protein"
      /note="self-assembling peptide"
      /organism="artificial sequences"

<400> 20
Trp Lys Trp Glu
1

<210> 21
<211> 4
<212> PRT
<213> artificial sequences

<220>
<221> SOURCE
<222> 1..4
<223> /mol_type="protein"
      /note="self-assembling peptide"
      /organism="artificial sequences"

<400> 21
Trp Glu Trp Arg
1

<210> 22
<211> 4
<212> PRT
<213> artificial sequences

<220>
<221> SOURCE
<222> 1..4
<223> /mol_type="protein"
      /note="self-assembling peptide"
      /organism="artificial sequences"

<400> 22
Trp Arg Trp Glu
1

<210> 23
<211> 4
<212> PRT
<213> artificial sequences

<220>
<221> SOURCE
<222> 1..4
<223> /mol_type="protein"
      /note="self-assembling peptide"
      /organism="artificial sequences"

<400> 23
Trp Lys Trp Arg
1

<210> 24
<211> 4
<212> PRT
<213> artificial sequences

<220>
<221> SOURCE
<222> 1..4
<223> /mol_type="protein"

/note="self-assembling peptide"
      /organism="artificial sequences"

<400> 24
Trp Arg Trp Lys
1

<210> 25
<211> 4
<212> PRT
<213> artificial sequences

<220>
<221> SOURCE
<222> 1..4
<223> /mol_type="protein"
      /note="self-assembling peptide"
      /organism="artificial sequences"

<400> 25
Trp Asp Trp Asp
1

<210> 26
<211> 4
<212> PRT
<213> artificial sequences

<220>
<221> SOURCE
<222> 1..4
<223> /mol_type="protein"
      /note="self-assembling peptide"
      /organism="artificial sequences"

<400> 26
Trp Asp Trp Glu
1

<210> 27
<211> 4
<212> PRT
<213> artificial sequences

<220>
<221> SOURCE
<222> 1..4
<223> /mol_type="protein"
      /note="self-assembling peptide"
      /organism="artificial sequences"

<400> 27
Trp Glu Trp Asp
1

<210> 28
<211> 4
<212> PRT
<213> artificial sequences

<220>
<221> SOURCE
<222> 1..4
<223> /mol_type="protein"
      /note="self-assembling peptide"

/organism="artificial sequences"

<400> 28
Trp Asp Trp Lys
1

<210> 29
<211> 4
<212> PRT
<213> artificial sequences

<220>
<221> SOURCE
<222> 1..4
<223> /mol_type="protein"
/note="self-assembling peptide"
/organism="artificial sequences"

<400> 29
Trp Lys Trp Asp
1

<210> 30
<211> 4
<212> PRT
<213> artificial sequences

<220>
<221> SOURCE
<222> 1..4
<223> /mol_type="protein"
/note="self-assembling peptide"
/organism="artificial sequences"

<400> 30
Trp Asp Trp Arg
1

<210> 31
<211> 4
<212> PRT
<213> artificial sequences

<220>
<221> SOURCE
<222> 1..4
<223> /mol_type="protein"
/note="self-assembling peptide"
/organism="artificial sequences"

<400> 31
Trp Arg Trp Asp
1

<210> 32
<211> 4
<212> PRT
<213> artificial sequences

<220>
<221> SOURCE
<222> 1..4
<223> /mol_type="protein"
/note="self-assembling peptide"
/organism="artificial sequences"

<400> 32
Ile Glu Ile Glu
1

<210> 33
<211> 4
<212> PRT
<213> artificial sequences

<220>
<221> SOURCE
<222> 1..4
<223> /mol_type="protein"
      /note="self-assembling peptide"
      /organism="artificial sequences"

<400> 33
Ile Glu Ile Lys
1

<210> 34
<211> 4
<212> PRT
<213> artificial sequences

<220>
<221> SOURCE
<222> 1..4
<223> /mol_type="protein"
      /note="self-assembling peptide"
      /organism="artificial sequences"

<400> 34
Ile Arg Ile Arg
1

<210> 35
<211> 4
<212> PRT
<213> artificial sequences

<220>
<221> SOURCE
<222> 1..4
<223> /mol_type="protein"
      /note="self-assembling peptide"
      /organism="artificial sequences"

<400> 35
Ile Lys Ile Glu
1

<210> 36
<211> 4
<212> PRT
<213> artificial sequences

<220>
<221> SOURCE
<222> 1..4
<223> /mol_type="protein"
      /note="self-assembling peptide"
      /organism="artificial sequences"

<400> 36
Ile Glu Ile Arg
1

<210> 37
<211> 4
<212> PRT
<213> artificial sequences

<220>
<221> SOURCE
<222> 1..4
<223> /mol_type="protein"
      /note="self-assembling peptide"
      /organism="artificial sequences"

<400> 37
Ile Arg Ile Glu
1

<210> 38
<211> 4
<212> PRT
<213> artificial sequences

<220>
<221> SOURCE
<222> 1..4
<223> /mol_type="protein"
      /note="self-assembling peptide"
      /organism="artificial sequences"

<400> 38
Ile Lys Ile Arg
1

<210> 39
<211> 4
<212> PRT
<213> artificial sequences

<220>
<221> SOURCE
<222> 1..4
<223> /mol_type="protein"
      /note="self-assembling peptide"
      /organism="artificial sequences"

<400> 39
Ile Arg Ile Lys
1

<210> 40
<211> 4
<212> PRT
<213> artificial sequences

<220>
<221> SOURCE
<222> 1..4
<223> /mol_type="protein"
      /note="self-assembling peptide"
      /organism="artificial sequences"

<400> 40

Ile Asp Ile Asp
1

<210> 41
<211> 4
<212> PRT
<213> artificial sequences

<220>
<221> SOURCE
<222> 1..4
<223> /mol_type="protein"
      /note="self-assembling peptide"
      /organism="artificial sequences"

<400> 41
Ile Asp Phe Glu
1

<210> 42
<211> 4
<212> PRT
<213> artificial sequences

<220>
<221> SOURCE
<222> 1..4
<223> /mol_type="protein"
      /note="self-assembling peptide"
      /organism="artificial sequences"

<400> 42
Ile Glu Ile Asp
1

<210> 43
<211> 4
<212> PRT
<213> artificial sequences

<220>
<221> SOURCE
<222> 1..4
<223> /mol_type="protein"
      /note="self-assembling peptide"
      /organism="artificial sequences"

<400> 43
Ile Asp Ile Lys
1

<210> 44
<211> 4
<212> PRT
<213> artificial sequences

<220>
<221> SOURCE
<222> 1..4
<223> /mol_type="protein"
      /note="self-assembling peptide"
      /organism="artificial sequences"

<400> 44
Ile Lys Ile Asp

1

<210> 45
<211> 4
<212> PRT
<213> artificial sequences

<220>
<221> SOURCE
<222> 1..4
<223> /mol_type="protein"
      /note="self-assembling peptide"
      /organism="artificial sequences"

<400> 45
Ile Asp Ile Arg
1

<210> 46
<211> 4
<212> PRT
<213> artificial sequences

<220>
<221> SOURCE
<222> 1..4
<223> /mol_type="protein"
      /note="self-assembling peptide"
      /organism="artificial sequences"

<400> 46
Ile Arg Ile Asp
1

<210> 47
<211> 4
<212> PRT
<213> artificial sequences

<220>
<221> SOURCE
<222> 1..4
<223> /mol_type="protein"
      /note="self-assembling peptide"
      /organism="artificial sequences"

<400> 47
Tyr Glu Tyr Glu
1

<210> 48
<211> 4
<212> PRT
<213> artificial sequences

<220>
<221> SOURCE
<222> 1..4
<223> /mol_type="protein"
      /note="self-assembling peptide"
      /organism="artificial sequences"

<400> 48
Tyr Arg Tyr Arg
1

<210> 49
<211> 4
<212> PRT
<213> artificial sequences

<220>
<221> SOURCE
<222> 1..4
<223> /mol_type="protein"
      /note="self-assembling peptide"
      /organism="artificial sequences"

<400> 49
Tyr Glu Tyr Lys
1

<210> 50
<211> 4
<212> PRT
<213> artificial sequences

<220>
<221> SOURCE
<222> 1..4
<223> /mol_type="protein"
      /note="self-assembling peptide"
      /organism="artificial sequences"

<400> 50
Tyr Lys Tyr Glu
1

<210> 51
<211> 4
<212> PRT
<213> artificial sequences

<220>
<221> SOURCE
<222> 1..4
<223> /mol_type="protein"
      /note="self-assembling peptide"
      /organism="artificial sequences"

<400> 51
Tyr Glu Tyr Arg
1

<210> 52
<211> 4
<212> PRT
<213> artificial sequences

<220>
<221> SOURCE
<222> 1..4
<223> /mol_type="protein"
      /note="self-assembling peptide"
      /organism="artificial sequences"

<400> 52
Tyr Arg Tyr Glu
1

<210> 53
<211> 4
<212> PRT
<213> artificial sequences

<220>
<221> SOURCE
<222> 1..4
<223> /mol_type="protein"
      /note="self-assembling peptide"
      /organism="artificial sequences"

<400> 53
Tyr Lys Tyr Arg
1

<210> 54
<211> 4
<212> PRT
<213> artificial sequences

<220>
<221> SOURCE
<222> 1..4
<223> /mol_type="protein"
      /note="self-assembling peptide"
      /organism="artificial sequences"

<400> 54
Tyr Arg Tyr Lys
1

<210> 55
<211> 4
<212> PRT
<213> artificial sequences

<220>
<221> SOURCE
<222> 1..4
<223> /mol_type="protein"
      /note="self-assembling peptide"
      /organism="artificial sequences"

<400> 55
Tyr Asp Tyr Asp
1

<210> 56
<211> 4
<212> PRT
<213> artificial sequences

<220>
<221> SOURCE
<222> 1..4
<223> /mol_type="protein"
      /note="self-assembling peptide"
      /organism="artificial sequences"

<400> 56
Tyr Asp Tyr Glu
1

<210> 57

<211> 4
<212> PRT
<213> artificial sequences

<220>
<221> SOURCE
<222> 1..4
<223> /mol_type="protein"
      /note="self-assembling peptide"
      /organism="artificial sequences"

<400> 57
Tyr Glu Tyr Asp
1

<210> 58
<211> 4
<212> PRT
<213> artificial sequences

<220>
<221> SOURCE
<222> 1..4
<223> /mol_type="protein"
      /note="self-assembling peptide"
      /organism="artificial sequences"

<400> 58
Tyr Asp Tyr Lys
1

<210> 59
<211> 4
<212> PRT
<213> artificial sequences

<220>
<221> SOURCE
<222> 1..4
<223> /mol_type="protein"
      /note="self-assembling peptide"
      /organism="artificial sequences"

<400> 59
Tyr Lys Tyr Asp
1

<210> 60
<211> 4
<212> PRT
<213> artificial sequences

<220>
<221> SOURCE
<222> 1..4
<223> /mol_type="protein"
      /note="self-assembling peptide"
      /organism="artificial sequences"

<400> 60
Tyr Asp Tyr Arg
1

<210> 61
<211> 4

<212> PRT
<213> artificial sequences

<220>
<221> SOURCE
<222> 1..4
<223> /mol_type="protein"
      /note="self-assembling peptide"
      /organism="artificial sequences"

<400> 61
Tyr Arg Tyr Asp
1

<210> 62
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 62
Xaa Glu Xaa Glu
1

<210> 63
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 63
Xaa Lys Xaa Lys
1

<210> 64
<211> 4
<212> PRT
<213> artificial sequences

<220>
<221> SOURCE
<222> 1..4
<223> /mol_type="protein"

/note="self-assembling peptide"
      /organism="artificial sequences"

<220>
<221> MOD_RES
<222> 1,3
<223> Nle

<400> 64
Xaa Arg Xaa Arg
1

<210> 65
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 65
Xaa Glu Xaa Lys
1

<210> 66
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 66
Xaa Lys Xaa Glu
1

<210> 67
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 67
Xaa Glu Xaa Arg
1

<210> 68
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 68
Xaa Arg Xaa Glu
1

<210> 69
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 69
Xaa Lys Xaa Arg
1

<210> 70
<211> 4
<212> PRT
<213> artificial sequences

<220>
<221> SOURCE
<222> 1..4
<223> /mol_type="protein"
      /note="self-assembling peptide"
      /organism="artificial sequences"

<220>

<221> MOD_RES
<222> 1,3
<223> Nle

<400> 70
Xaa Arg Xaa Lys
1

<210> 71
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 71
Xaa Asp Xaa Asp
1

<210> 72
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 72
Xaa Asp Xaa Glu
1

<210> 73
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3

<223> Nle

<400> 73
Xaa Glu Xaa Asp
1

<210> 74
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 74
Xaa Asp Xaa Lys
1

<210> 75
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 75
Xaa Lys Xaa Asp
1

<210> 76
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 76
Xaa Asp Xaa Arg
1

<210> 77
<211> 4
<212> PRT
<213> artificial sequences

<220>
<221> SOURCE
<222> 1..4
<223> /mol_type="protein"
      /note="self-assembling peptide"
      /organism="artificial sequences"

<220>
<221> MOD_RES
<222> 1,3
<223> Nle

<400> 77
Xaa Arg Xaa Asp
1

<210> 78
<211> 8
<212> PRT
<213> artificial sequences

<220>
<221> SOURCE
<222> 1..8
<223> /mol_type="protein"
      /note="self-assembling peptide"
      /organism="artificial sequences"

<400> 78
Phe Glu Phe Lys Phe Glu Phe Lys
1               5

<210> 79
<211> 8
<212> PRT
<213> artificial sequences

<220>
<221> SOURCE
<222> 1..8
<223> /mol_type="protein"
      /note="self-assembling peptide"
      /organism="artificial sequences"

<400> 79
Phe Glu Phe Glu Phe Lys Phe Lys
1               5

<210> 80
<211> 8
<212> PRT
<213> artificial sequences

<220>
<221> SOURCE
<222> 1..8

<223> /mol_type="protein"
      /note="self-assembling peptide"
      /organism="artificial sequences"

<400> 80
Phe Asp Phe Lys Phe Asp Phe Lys
1               5

<210> 81
<211> 8
<212> PRT
<213> artificial sequences

<220>
<221> SOURCE
<222> 1..8
<223> /mol_type="protein"
      /note="self-assembling peptide"
      /organism="artificial sequences"

<400> 81
Phe Asp Phe Asp Phe Lys Phe Lys
1               5

<210> 82
<211> 8
<212> PRT
<213> artificial sequences

<220>
<221> SOURCE
<222> 1..8
<223> /mol_type="protein"
      /note="self-assembling peptide"
      /organism="artificial sequences"

<400> 82
Phe Glu Phe Arg Phe Glu Phe Arg
1               5

<210> 83
<211> 8
<212> PRT
<213> artificial sequences

<220>
<221> SOURCE
<222> 1..8
<223> /mol_type="protein"
      /note="self-assembling peptide"
      /organism="artificial sequences"

<400> 83
Phe Glu Phe Glu Phe Arg Phe Arg
1               5

<210> 84
<211> 8
<212> PRT
<213> artificial sequences

<220>
<221> SOURCE
<222> 1..8
<223> /mol_type="protein"

/note="self-assembling peptide"
      /organism="artificial sequences"

<400> 84
Tyr Asp Tyr Lys Tyr Asp Tyr Lys
1               5

<210> 85
<211> 8
<212> PRT
<213> artificial sequences

<220>
<221> SOURCE
<222> 1..8
<223> /mol_type="protein"
      /note="self-assembling peptide"
      /organism="artificial sequences"

<400> 85
Tyr Asp Tyr Asp Tyr Lys Tyr Lys
1               5

<210> 86
<211> 8
<212> PRT
<213> artificial sequences

<220>
<221> SOURCE
<222> 1..8
<223> /mol_type="protein"
      /note="self-assembling peptide"
      /organism="artificial sequences"

<400> 86
Tyr Glu Tyr Arg Tyr Glu Tyr Arg
1               5

<210> 87
<211> 8
<212> PRT
<213> artificial sequences

<220>
<221> SOURCE
<222> 1..8
<223> /mol_type="protein"
      /note="self-assembling peptide"
      /organism="artificial sequences"

<400> 87
Tyr Glu Tyr Lys Tyr Glu Tyr Lys
1               5

<210> 88
<211> 8
<212> PRT
<213> artificial sequences

<220>
<221> SOURCE
<222> 1..8
<223> /mol_type="protein"
      /note="self-assembling peptide"

/organism="artificial sequences"

<400> 88
Tyr Glu Tyr Glu Tyr Lys Tyr Lys
1               5

<210> 89
<211> 8
<212> PRT
<213> artificial sequences

<220>
<221> SOURCE
<222> 1..8
<223> /mol_type="protein"
      /note="self-assembling peptide"
      /organism="artificial sequences"

<400> 89
Trp Glu Trp Lys Trp Glu Trp Lys
1               5

<210> 90
<211> 8
<212> PRT
<213> artificial sequences

<220>
<221> SOURCE
<222> 1..8
<223> /mol_type="protein"
      /note="self-assembling peptide"
      /organism="artificial sequences"

<400> 90
Trp Glu Trp Glu Trp Lys Trp Lys
1               5

<210> 91
<211> 8
<212> PRT
<213> artificial sequences

<220>
<221> SOURCE
<222> 1..8
<223> /mol_type="protein"
      /note="self-assembling peptide"
      /organism="artificial sequences"

<400> 91
Trp Asp Trp Lys Trp Asp Trp Lys
1               5

<210> 92
<211> 8
<212> PRT
<213> artificial sequences

<220>
<221> SOURCE
<222> 1..8
<223> /mol_type="protein"
      /note="self-assembling peptide"
      /organism="artificial sequences"

<400> 92
Trp Asp Trp Asp Trp Lys Trp Lys
1               5

<210> 93
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 93
Phe Glu Phe Lys Phe Glu Phe Lys
1               5

<210> 94
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 94
Phe Glu Phe Glu Phe Lys Phe Lys
1               5

<210> 95
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 95

Phe Asp Phe Lys Phe Asp Phe Lys
1               5

<210> 96
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 96
Phe Asp Phe Asp Phe Lys Phe Lys
1               5

<210> 97
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 97
Phe Glu Phe Arg Phe Glu Phe Arg
1               5

<210> 98
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 98
Phe Glu Phe Glu Phe Arg Phe Arg
1               5

<210> 99
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 99
Tyr Asp Tyr Lys Tyr Asp Tyr Lys
1               5

<210> 100
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 100
Tyr Asp Tyr Asp Tyr Lys Tyr Lys
1               5

<210> 101
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 101
Tyr Glu Tyr Arg Tyr Glu Tyr Arg
1               5

<210> 102

<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 102
Tyr Glu Tyr Lys Tyr Glu Tyr Lys
1               5

<210> 103
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 103
Tyr Glu Tyr Glu Tyr Lys Tyr Lys
1               5

<210> 104
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 104
Trp Glu Trp Lys Trp Glu Trp Lys
1               5

<210> 105
<211> 8
<212> PRT

<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 105
Trp Glu Trp Glu Trp Lys Trp Lys
1               5

<210> 106
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 106
Trp Asp Trp Lys Trp Asp Trp Lys
1               5

<210> 107
<211> 8
<212> PRT
<213> artificial sequences

<220>
<221> SOURCE
<222> 1..8
<223> /mol_type="protein"
      /note="self-assembling peptide"
      /organism="artificial sequences"

<220>
<221> MOD_RES
<222> 8
<223> AMIDATION

<400> 107
Trp Asp Trp Asp Trp Lys Trp Lys
1               5

<210> 108
<211> 4
<212> PRT
<213> artificial sequences

<220>
<221> SOURCE
<222> 1..4
<223> /mol_type="protein"
      /note="bioactive sequence"
      /organism="artificial sequences"

<400> 108
Arg Gly Asp Ser
1

<210> 109
<211> 5
<212> PRT
<213> artificial sequences

<220>
<221> SOURCE
<222> 1..5
<223> /mol_type="protein"
      /note="bioactive sequence"
      /organism="artificial sequences"

<400> 109
Gly Arg Gly Asp Ser
1               5

<210> 110
<211> 5
<212> PRT
<213> artificial sequences

<220>
<221> SOURCE
<222> 1..5
<223> /mol_type="protein"
      /note="bioactive sequence"
      /organism="artificial sequences"

<400> 110
Gly Arg Gly Asp Tyr
1               5

<210> 111
<211> 5
<212> PRT
<213> artificial sequences

<220>
<221> SOURCE
<222> 1..5
<223> /mol_type="protein"
      /note="bioactive sequence"
      /organism="artificial sequences"

<400> 111
Gly Arg Gly Asp Phe
1               5

<210> 112
<211> 5
<212> PRT
<213> artificial sequences

<220>

<221> SOURCE
<222> 1..5
<223> /mol_type="protein"
      /note="bioactive sequence"
      /organism="artificial sequences"

<400> 112
Tyr Gly Arg Gly Asp
1               5

<210> 113
<211> 6
<212> PRT
<213> artificial sequences

<220>
<221> SOURCE
<222> 1..6
<223> /mol_type="protein"
      /note="bioactive sequence"
      /organism="artificial sequences"

<400> 113
Gly Arg Gly Asp Ser Tyr
1               5

<210> 114
<211> 6
<212> PRT
<213> artificial sequences

<220>
<221> SOURCE
<222> 1..6
<223> /mol_type="protein"
      /note="bioactive sequence"
      /organism="artificial sequences"

<400> 114
Gly Arg Gly Asp Ser Pro
1               5

<210> 115
<211> 7
<212> PRT
<213> artificial sequences

<220>
<221> SOURCE
<222> 1..7
<223> /mol_type="protein"
      /note="bioactive sequence"
      /organism="artificial sequences"

<400> 115
Gly Arg Gly Asp Ser Pro Lys
1               5

<210> 116
<211> 5
<212> PRT
<213> artificial sequences

<220>
<221> SOURCE

<222> 1..5
<223> /mol_type="protein"
      /note="bioactive sequence"
      /organism="artificial sequences"

<400> 116
Tyr Arg Gly Asp Ser
1               5

<210> 117
<211> 6
<212> PRT
<213> artificial sequences

<220>
<221> SOURCE
<222> 1..6
<223> /mol_type="protein"
      /note="bioactive sequence"
      /organism="artificial sequences"

<400> 117
Gly Arg Gly Glu Thr Pro
1               5

<210> 118
<211> 6
<212> PRT
<213> artificial sequences

<220>
<221> SOURCE
<222> 1..6
<223> /mol_type="protein"
      /note="bioactive sequence"
      /organism="artificial sequences"

<400> 118
Gly Arg Gly Glu Ser Pro
1               5

<210> 119
<211> 6
<212> PRT
<213> artificial sequences

<220>
<221> SOURCE
<222> 1..6
<223> /mol_type="protein"
      /note="bioactive sequence"
      /organism="artificial sequences"

<400> 119
Gly Arg Gly Asp Thr Pro
1               5

<210> 120
<211> 4
<212> PRT
<213> artificial sequences

<220>
<221> SOURCE
<222> 1..4

<223> /mol_type="protein"
      /note="bioactive sequence"
      /organism="artificial sequences"

<400> 120
Ser Asp Pro Lys
1

<210> 121
<211> 6
<212> PRT
<213> artificial sequences

<220>
<221> SOURCE
<222> 1..6
<223> /mol_type="protein"
      /note="bioactive sequence"
      /organism="artificial sequences"

<400> 121
Gly Arg Gly Asp Ser Pro
1               5

<210> 122
<211> 5
<212> PRT
<213> artificial sequences

<220>
<221> SOURCE
<222> 1..5
<223> /mol_type="protein"
      /note="bioactive sequence"
      /organism="artificial sequences"

<400> 122
Gly Arg Gly Asp Lys
1               5

<210> 123
<211> 7
<212> PRT
<213> artificial sequences

<220>
<221> SOURCE
<222> 1..7
<223> /mol_type="protein"
      /note="bioactive sequence"
      /organism="artificial sequences"

<400> 123
Gly Arg Ala Asp Ser Pro Lys
1               5

<210> 124
<211> 8
<212> PRT
<213> artificial sequences

<220>
<221> SOURCE
<222> 1..8
<223> /mol_type="protein"

/note="bioactive sequence"
      /organism="artificial sequences"

<400> 124
Gly Gly Gly Gly Arg Gly Asp Ser
1               5

<210> 125
<211> 6
<212> PRT
<213> artificial sequences

<220>
<221> SOURCE
<222> 1..6
<223> /mol_type="protein"
      /note="bioactive sequence"
      /organism="artificial sequences"

<400> 125
Gly Arg Gly Asp Asn Pro
1               5

<210> 126
<211> 5
<212> PRT
<213> artificial sequences

<220>
<221> SOURCE
<222> 1..5
<223> /mol_type="protein"
      /note="bioactive sequence"
      /organism="artificial sequences"

<400> 126
Arg Gly Asp Tyr Lys
1               5

<210> 127
<211> 5
<212> PRT
<213> artificial sequences

<220>
<221> SOURCE
<222> 1..5
<223> /mol_type="protein"
      /note="bioactive sequence"
      /organism="artificial sequences"

<400> 127
Arg Gly Asp Phe Lys
1               5

<210> 128
<211> 4
<212> PRT
<213> artificial sequences

<220>
<221> SOURCE
<222> 1..4
<223> /mol_type="protein"
      /note="bioactive sequence"

/organism="artificial sequences"

<400> 128
Arg Glu Asp Val
1

<210> 129
<211> 4
<212> PRT
<213> artificial sequences

<220>
<221> SOURCE
<222> 1..4
<223> /mol_type="protein"
      /note="bioactive sequence"
      /organism="artificial sequences"

<400> 129
Arg Gly Asp Val
1

<210> 130
<211> 5
<212> PRT
<213> artificial sequences

<220>
<221> SOURCE
<222> 1..5
<223> /mol_type="protein"
      /note="bioactive sequence"
      /organism="artificial sequences"

<400> 130
Leu Arg Gly Asp Asn
1               5

<210> 131
<211> 5
<212> PRT
<213> artificial sequences

<220>
<221> SOURCE
<222> 1..5
<223> /mol_type="protein"
      /note="bioactive sequence"
      /organism="artificial sequences"

<400> 131
Ile Lys Val Ala Val
1               5

<210> 132
<211> 5
<212> PRT
<213> artificial sequences

<220>
<221> SOURCE
<222> 1..5
<223> /mol_type="protein"
      /note="bioactive sequence"
      /organism="artificial sequences"

<400> 132
Tyr Ile Gly Ser Arg
1               5

<210> 133
<211> 5
<212> PRT
<213> artificial sequences

<220>
<221> SOURCE
<222> 1..5
<223> /mol_type="protein"
      /note="bioactive sequence"
      /organism="artificial sequences"

<400> 133
Pro Asp Ser Gly Arg
1               5

<210> 134
<211> 10
<212> PRT
<213> artificial sequences

<220>
<221> SOURCE
<222> 1..10
<223> /mol_type="protein"
      /note="bioactive sequence"
      /organism="artificial sequences"

<400> 134
Arg Asn Ala Ile Glu Ile Ile Lys Asp Ala
1               5                   10

<210> 135
<211> 4
<212> PRT
<213> artificial sequences

<220>
<221> SOURCE
<222> 1..4
<223> /mol_type="protein"
      /note="bioactive sequence"
      /organism="artificial sequences"

<400> 135
Arg Gly Asp Thr
1

<210> 136
<211> 4
<212> PRT
<213> artificial sequences

<220>
<221> SOURCE
<222> 1..4
<223> /mol_type="protein"
      /note="bioactive sequence"
      /organism="artificial sequences"

<400> 136
Asp Gly Glu Ala
1

<210> 137
<211> 7
<212> PRT
<213> artificial sequences

<220>
<221> SOURCE
<222> 1..7
<223> /mol_type="protein"
      /note="bioactive sequence"
      /organism="artificial sequences"

<400> 137
Gln His Arg Glu Asp Gly Ser
1               5

<210> 138
<211> 5
<212> PRT
<213> artificial sequences

<220>
<221> SOURCE
<222> 1..5
<223> /mol_type="protein"
      /note="bioactive sequence"
      /organism="artificial sequences"

<400> 138
Arg Gly Asp Trp Pro
1               5

<210> 139
<211> 5
<212> PRT
<213> artificial sequences

<220>
<221> SOURCE
<222> 1..5
<223> /mol_type="protein"
      /note="bioactive sequence"
      /organism="artificial sequences"

<220>
<221> MOD_RES
<222> 1
<223> Har

<400> 139
Xaa Gly Asp Trp Pro
1               5

## Description

The present invention relates to a process for preparing self-assembling peptides in liquid phase. These self-assembling peptides can be covalently linked to thermoresponsive or biodegradable polymers, to another peptide comprising another self-assembling peptide moiety, or a bioactive sequence. The self-assembling peptide alone or in combination with a peptide-peptide or polymer-peptide conjugate can be used as a support for cell cultures or for the encapsulation of drugs or cells. Therefore, the materials provided by the processes of the present invention can be advantageously employed in areas such as production of vaccines for human and veterinary use, culturing cell layers for cell therapy, tissue engineering and tissue repair as well as in drug delivery systems.

### Abbreviations

In the text of the present application, the nomenclature of amino acids and of peptides is used according to "Nomenclature and symbolism for amino acids and peptides", Pure & Appl. Chem., Vol. 56, No. 5, pp. 595-624, 1984, if not stated otherwise. The following abbreviations have the meaning as given in the following list :
- Alloc: allyloxycarbonyl
- Boc: *tert*-butoxycarbonyl
- DCC: *N,N'*-dicyclohexylcarbodiimide
- DCM: dichloromethane
- DCU: *N,N'*-dicyclohexylurea
- DIPEA: *N,N*-diisopropylethylamine
- DMA: *N,N*-dimethylacetamide
- DMAPA: *N,N*-dimethylaminopropylamide
- DMF: *N,N*-dimethylformamide
- EDC: 1-ethyl-3-(3-dimethylaminopropyl)carbodiimide
- EtOAc: ethyl acetate
- Fmoc: fluorenyl-9-methoxycarbonyl
- h: hour(s)
- hArg, hR: homoarginine
- HOBt: 1-hydroxybenzotriazole
- HONb: *N*-hydroxy-5-norbornene-2,3-dicarboximide
- HOSu: *N*-hydroxysuccinimide
- HPLC: high-performance liquid chromatography
- HPMA: hydroxypropyl methacrylamide
- IBCF: isobutylchloroformate
- IPE: diisopropyl ether
- iPrOAc: isopropyl acetate
- KOH: potassium hydroxide
- LPPS: liquid phase peptide synthesis
- MeCN: acetonitrile
- min: minute(s)
- MTBE: methyl-t-buthyl ether
- NMP: *N*-methyl-2-pyrrolidone
- NMR: nuclear magnetic resonance spectroscopy
- Nle: norleucine
- O*t*Bu: *tert*-butoxy
- PG: protective group
- PivCl: pivaloyl chloride
- *p*TosOH: *p*-toluene sulfonic acid
- SPPS: solid phase peptide synthesis
- *t*Bu: *tert*-butyl
- TEA: triethylamine
- TFA: trifluoroacetic acid
- TMA: *N*-trimethylsilylacetamide
- t_{R}: retention time
- Trt: trityl
- UV: ultraviolet
- Z: benzyloxycarbonyl

Amino acids are designated by the commonly used one-letter or three-letter codes.

Peptides that self-assemble into a macroscopic structure when present in unmodified form are known in the prior art. For instance, S. Zhang et al. (Biotechnology Advances, vol. 20, p. 321-339, 2002) disclose several types of such self-assembling peptides.

M. R. Caplan et al. (Biomaterials, Vol. 23, p. 219-227, 2002) describe a class of self-assembling peptides capable of forming β-strands. These peptides have a repeated sequence of amino acid residues with the type : hydrophobic/negatively-charged/hydrophobic/positively-charged.

Another example is given by US7713923, which discloses self-assembling peptides comprising a first domain that mediates self-assembly into a macroscopic structure and a second domain comprising biologically active peptide motif.

The synthesis of such self-assembling peptides is typically carried out by a solid phase peptide synthesis (SPPS). Industrial peptide manufacturing employing SPPS is relatively cost- and material intensive, which leads to high manufacturing costs of the target peptides. In addition, these peptides often require an additional purification by preparative high-performance liquid chromatography (HPLC), which further increases their manufacturing costs. Consequently, the biomaterials comprising such peptides are also relatively expensive and their practical use is limited to some extent for this reason.

Thus, there is a significant demand for a cost- and material efficient process allowing a preparation of self-assembling peptides in a sufficient purity on industrial scale. Such process would allow a cost-efficient manufacturing of biomaterials comprising these peptides and therefore provide these biomaterials for a wide range of applications.

According to the present invention a process for preparing a self-assembling peptide moiety comprising an amino acid sequence (I)

H-Xaa1-Xaa5-Xaa3-Xaa6-R1 (I),

wherein R1 is selected from OH and NH₂
and of a self-assembling peptide moiety comprising an amino acid sequence (II)

H-Xaa1-Xaa2-Xaa3-Xaa4-Xaa1-Xaa5-Xaa3-Xaa6-R1 (II),

wherein R1 is selected from OH and NH₂,
is disclosed by a highly convergent liquid phase peptide synthesis (LPPS) to be preferably performed on industrial scale. The isolated peptide moiety is sufficiently pure for the subsequent use and does not require any purification by preparative HPLC.

Xaa1 and Xaa3 are residues of hydrophobic amino acids and are preferably selected from the group consisting of phenylalanine, tryptophan, histidine, tyrosine, isoleucine, alanine, leucine and valine. Xaa2, Xaa4, Xaa5 and Xaa6 are residues of charged amino acids and are preferably selected from the group consisting of arginine, aspartic acid, glutamic acid and lysine.

The process of the present invention is carried out in liquid phase and comprises the following peptide coupling steps a)-c) for amino acid sequence (I) :
a) coupling of PG1-Xaa1-OH and H-Xaa5-OH to form PG1-Xaa1-Xaa5-OH ;
b) coupling of PG1-Xaa3-OH and H-Xaa6-R1 to form PG1-Xaa3-Xaa6-R1 ; and
c) coupling of PG1-Xaa1-Xaa5-OH and H-Xaa3-Xaa6-R1 to form PG1-Xaa1-Xaa5-Xaa3-Xaa6-R1 ;
to form the peptide moiety comprising the amino acid sequence (I).

Yet according to another aspect of the present invention, the process of the present invention is carried out in liquid phase and comprises the following peptide coupling steps aa)-gg) for amino acid sequence (II) :
aa) coupling of PG1-Xaa1-OH and H-Xaa2-OH to form PG1-Xaa1-Xaa2-OH ;
bb) coupling of PG1-Xaa3-OH and H-Xaa4-OH to form PG1-Xaa3-Xaa4-OH ;
cc) coupling of PG1-Xaal-Xaa2-OH and H-Xaa3-Xaa4-OH to form PG1-Xaa1-Xaa2-Xaa3-Xaa4-OH;
dd) coupling of PG1-Xaa1-OH and H-Xaa5-OH to form PG1-Xaa1-Xaa5-OH ;
ee) coupling of PG1-Xaa3-OH and H-Xaa6-R1 to form PG1-Xaa3-Xaa6-R1 ;
ff) coupling of PG1-Xaa1-Xaa5-OH and H-Xaa3-Xaa6-R1 to form PG1-Xaa1-Xaa5-Xaa3-Xaa6-R1 ;
gg) coupling of PG1-Xaa1-Xaa2-Xaa3-Xaa4-OH and H-Xaa1-Xaa5-Xaa3-Xaa6-R1 to form the peptide moiety comprising the amino acid sequence (II).

Thus, in some embodiments of the present invention the peptide moiety comprising the amino acid sequence (I) can serve as an intermediate in the process for preparing the peptide moiety comprising the amino acid sequence (II). Consequently, in these particular embodiments the peptide coupling steps a)-c) correspond to the peptide coupling steps dd)-ff).

Depending on the choice of amino acid residues Xaa1 to Xaa6 the sequence (II) can be symmetrical or asymmetrical. Thus, the process of the present invention is suitable for the preparation of a wide variety of self-assembling peptides.

In a preferred embodiment the coupling steps a)-c) and aa)-gg) employ persilylated amino acids or persilylated peptides, such as for instance persilylated dipeptides or persilylated tetrapeptides as intermediates.

In one of the preferred embodiments of the present invention the amino acid residues Xaa2 and Xaa5 are identical residues. The residues Xaa4 and Xaa6 are also identical residues. Thus, the amino acid sequence (II) is symmetrical.

In another preferred embodiment of the present invention the amino acid residues Xaa2 and Xaa4 are identical. The amino acid residues Xaa5 and Xaa6 are also identical residues. Thus, in this particular embodiment the sequence (II) is asymmetrical.

These self-assembling peptides prepared by the process of the present invention can be covalently linked directly or through a spacer to :
- peptides comprising a bioactive peptide sequence,
- other self-assembling peptides,
- biodegradable, thermoresponsive, pH sensitive polymer, or
- a combination thereof.

### Detailed description

One aspect of the present invention relates to a process for preparing a peptide moiety comprising an amino acid sequence (I)

H-Xaa1-Xaa5-Xaa3-Xaa6-R1 (I),

wherein R1 is selected from OH and NH₂
whereby Xaa1 and Xaa3 are residues of hydrophobic amino acids. Preferably Xaa1 and Xaa3 are selected from the group consisting of phenylalanine, tryptophan, histidine, tyrosine, leucine, isoleucine, norleucine, alanine and valine. Xaa5 and Xaa6 are residues of charged amino acids and are preferably selected from the group consisting of arginine, aspartic acid, glutamic acid and lysine.

The corresponding process is carried out in liquid phase and comprises the following peptide coupling steps a)-c) :
a) coupling of PG1-Xaa1-OH and H-Xaa5-OH to form PG1-Xaa1-Xaa5-OH ;
b) coupling of PG1-Xaa3-OH and H-Xaa6-R1 to form PG1-Xaa3-Xaa6-R1 ;
   and
c) coupling of PG1-Xaa1-Xaa5-OH and H-Xaa3-Xaa6-R1 to form PG1-Xaa1-Xaa5-Xaa3-Xaa6-R1 ;
   to form the peptide moiety comprising the amino acid sequence (I),
   whereby PG1 is a *N*-terminal protective group.

Another aspect of the present invention relates to a process for preparing a peptide moiety comprising an amino acid sequence (II)

H-Xaa1-Xaa2-Xaa3-Xaa4-Xaa1-Xaa5-Xaa3-Xaa6-R1 (II),

wherein R1 is selected from OH and NH₂,
whereby Xaa1 and Xaa3 are residues of hydrophobic amino acids. Preferably Xaa1 and Xaa3 are selected from the group consisting of phenylalanine, tryptophan, histidine, tyrosine, leucine, isoleucine, norleucine, alanine and valine. Xaa2, Xaa4, Xaa5 and Xaa6 are residues of charged amino acids and are preferably selected from the group consisting of arginine, aspartic acid, glutamic acid and lysine.

The process is carried out in liquid phase and comprises the following peptide coupling steps aa)-gg) :
aa) coupling of PG1-Xaa1-OH and H-Xaa2-OH to form PG1-Xaa1-Xaa2-OH ;
bb) coupling of PG1-Xaa3-OH and H-Xaa4-OH to form PG1-Xaa3-Xaa4-OH ;
cc) coupling of PG1-Xaa1-Xaa2-OH and H-Xaa3-Xaa4-OH to form PG1-Xaa1-Xaa2-Xaa3-Xaa4-OH;
dd) coupling of PG1-Xaa1-OH and H-Xaa5-OH to form PG1-Xaa1-Xaa5-OH ; ee) coupling of PG1-Xaa3-OH and H-Xaa6-R1 to form PG1-Xaa3-Xaa6-R1 ; ff) coupling of PG1-Xaa1-Xaa5-OH and H-Xaa3-Xaa6-R1 to form PG1-Xaa1-Xaa5-Xaa3-Xaa6-R1 ;
gg) coupling of PG1-Xaa1-Xaa2-Xaa3-Xaa4-OH and H-Xaa1-Xaa5-Xaa3-Xaa6-R1 to form the peptide moiety comprising the amino acid sequence (II),
whereby PG1 is a *N*-terminal protective group.

As used herein, the term "amino acid" (Xaa) is intended to denote any compound having at least one primary or secondary amino group, preferably at least one primary amino group and at least one carboxylic acid group. The amino acids of the present invention can be naturally occurring or synthetic. The naturally occurring amino acids, with exception of glycine, contain a chiral carbon atom. Unless otherwise specifically indicated, the compounds containing natural amino acids with the L-configuration are preferred, whereby proteinogenic amino acids are particularly preferred.

In the text of the present application the term "hydrophobic amino acid" refers to an amino acid having log P value (partition coefficient) higher than -3.0. Thus, hydrophobic amino acids include but are not limited to alanine, cysteine, phenylalanine, histidine, isoleucine, leucine, methionine, norleucine, proline, tryptophan, tyrosine and valine.

*n*-Octanol-water partition coefficient P of an amino acid is defined as ratio of concentrations (mol/volume) of an amino acid in *n*-octanol and in water. Suitable methods for the measurement of *n*-octanol-water coefficients are, for instance described in the publication "Octanol-Water Partition Coefficients : Fundamentals and Physical Chemistry", John Wiley and Sons Ltd., 1997, ISBN : 0-417-97397 1.

Both solvents are mutually saturated before the measurement. At equilibrium the *n*-octanol phase contains 2.3 mol/l of water and the aqueous phase contains 4.5 x 10⁻³ mol/l of *n*-octanol. The measurement is carried out at the isoelectric point of the amino acid at temperature of 25°C.

The *n*-octanol-water coefficient P of an amino acid is preferably determined by the shake-flask method, which is, for example, described in the review of J. Sangster (J. Phys. Chem. Ref. Data 18, 3, pp. 1111-1227, 1989). The measurement is carried out under the conditions described by T. Fujita et al (J. Am. Chem. Soc. 86, pp. 5175-5180, 1964).

The concentration of the amino acid in the system should be less than 0.01 mol/l in any single phase. Very pure *n*-octanol and water must be used for the measurement. The system, usually in a separator funnel or similar device, is shaken gently until equilibrium is achieved (0.5 hours to 3 days). The system is then centrifuged to separate the two phases and break any emulsions.

Subsequently, both *n*-octanol phase and the aqueous phase are analysed to achieve mass balance whereby an HPLC instrument equipped with a suitable reversed-phase column is employed for the analysis.

Alternatively, the *n*-octanol-water coefficient of an amino acid can be evaluated by using the ALOGPS 2.1 software by I. V. Tetko et al. (J. Comput. Aid. Mol. Des. 19, pp. 453-63, 2005 and J. Chem. Inf. Comput. Sci. 41, pp. 1488-1493, 2001). The software is available from the Virtual Computational Chemistry Laboratory (VCCLAB, http://www.vcclab.org).

In the text of the present application the term "charged amino acids" refers to positively charged amino acids and negatively charged amino acids. A positively charged amino acid has log P value lower or equal to -3.0 and isoelectric point pI above 9.0. Thus, positively charged amino acids include but are not limited to arginine, homoarginine, ornithine, citrulline and lysine. A negatively charged amino acid has log P value lower or equal to -3.0 and isoelectric point pI lower than 4.0. Negatively charged amino acids include but are not limited to glutamic acid and aspartic acid.

Isoelectric point pI is the pH at which an amino acid carries no net electrical charge. Isoelectric point pI of an amino acid can be determined experimentally by using volumetric titration at 25°C as known in the prior art. Alternatively, pI can be determined by capillary isoelectric focusing, which can be carried out on a commercially available capillary electrophoresis system such as Agilent capillary electrophoresis system Agilent 7100.

A peptide is a compound produced by amide formation between a carboxylic acid group of one amino acid and an amino group of another. The amide bond in peptides is called peptide bond. As used herein, the term "peptide" comprises peptides and peptide analogues. Peptide analogues comprise natural amino acids and non-natural amino acids. They can also comprise modifications such as glycosylations. The peptides or peptide analogues can also comprise unnatural amino acids and amino acid mimetics that function in a manner similar to the naturally occurring amino acids. The peptides may also be formed from amino acids analogues that have modified peptide backbones. Peptide analogues usually include at least one bond in the peptide sequence which is different from an amide bond, such as urethane, urea, ester or thioester bond. Peptides or peptide analogues according to the present invention can be linear, cyclic or branched and are preferably linear.

In a preferred embodiment of the present invention the peptide moiety comprising an amino acid sequence (I) or an amino acid sequence (II) consists of 4 to 20 amino acid residues. However, in a particularly preferred embodiment, the peptide moiety comprising an amino acid sequence (I) is a tetrapeptide having amino acid sequence (I). In another particularly preferred embodiment, the peptide moiety comprising an amino acid sequence (II) is an octapeptide having amino acid sequence (II).

In one embodiment of the present invention the carboxylic acid group (C-terminus) of the amino acid residue Xaa6 of sequences (I) and (II) does not carry any protective group. In another embodiment of the present invention the C-terminus of the amino acid residue Xaa6 of sequences (I) and (II) is protected as an ester, preferably as ethyl ester.

Protective groups (PGs) and typical reaction conditions and reagents for introducing and cleaving PGs, which are conventionally used in the process of the present invention, are known in the prior art and described in e.g. T. W. Greene, P. G. M. Wuts "Greene's Protective Groups in Organic Synthesis", John Wiley & Sons, Inc., 2006 ; and in P. Lloyd-Williams, F. Albericio, E. Giralt, "Chemical Approaches to the Synthesis of Peptides and Proteins", CRC Press LLC, Boca Raton, Florida, 1997. Principles of peptide synthesis are also described in Sewald and Jakube : "Peptides : Chemistry and Biology", 2nd edition, Wiley-VCH, 2009.

Preferably, side chains of amino acid residues having substituents such as carboxylic acid group, amino group or imidazolyl group are protected with suitable PGs during the process of the present invention. In a particularly preferred embodiment the side chains of aspartic acid and glutamic acid residues are protected with ester type protective groups, the side chains of lysine residues are protected with carbamate type protective groups. The side chains of histidine residues can be protected as trityl (Trt), *tert*-butoxycarbonyl (Boc) or benzyloxycarbonyl (Z). The side chain of tyrosine residues are either not protected or protected as ester or ether. Side chains of arginine, tyrosine and tryptophan residues are preferably not protected during the process of the present invention.

Preferably, PG1 is a carbamate type protective group.

A carbamate type protective group suitable for the process of the present invention can be for instance Z, Boc, fluorenyl-9-methoxycarbonyl (Fmoc) or allyloxycarbonyl (Alloc). A suitable ester type protective group can be selected from protective groups such as methyl ester, ethyl ester, benzyl ester or *tert*-butyl ester.

In a particularly preferred embodiment of the present invention the side chains of aspartic acid and glutamic acid residues are protected as *tert*-butyl esters and the side chains of lysine residues are protected with at least one Boc protective group. Thus, all PGs of side chains can be preferably removed under acidic conditions in a single step.

In a preferred embodiment the *N*-terminal protective group PG1 is orthogonal to protective groups of the side chains and is stable under strongly acidic conditions. Preferably, PG1 is Z and is therefore cleavable under reducing conditions. In yet another preferred embodiment PG1 is Fmoc.

A solvent such as acetonitrile (MeCN), ethyl acetate (EtOAc), isopropyl acetate (iPrOAc), dichloromethane (DCM), *N,N-*dimethylacetamide (DMA), *N,N*-dimethylformamide (DMF) or *N*-methyl-2-pyrrolidone (NMP) can be employed as a reaction solvent in the peptide coupling steps a)-c) and aa)-gg). Preferably, the reaction solvent used in the peptide coupling steps a)-c) and aa)-gg) is substantially water-free and contains less than 1 wt.- % water, more preferred less than 0.1 wt.- % water, even more preferred less than 0.01 wt.- % water and particularly preferred less than 0.001 wt.- % water. The water content in the reaction solvent can be determined by Karl Fischer titration according to the standard test method ASTM E203-08.

The peptide coupling steps a)-c) and aa)-gg) are carried out upon using one or more coupling reagents, preferably in the presence of one or more coupling additives, and preferably in the presence of one or more tertiary bases.

The coupling reagents and coupling additives used for the peptide coupling steps a)-c) and aa)-gg) are chosen in such a way that they preferably do not lead to desilylation of persilylated amino acids or persilylated peptides employed in one of preferred embodiments and no substantial epimerisation of the stereogenic centre adjacent to the activated carboxylic acid group takes place. Preferred coupling reagents are either carbodiimide coupling reagents such as *N,N'*-dicyclohexylcarbodiimide (DCC) and 1-ethyl-3-(3-dimethylaminopropyl) carbodiimide (EDC), or isobutylchloroformate (IBCF) and pivaloyl chloride (PivCl), whereby PivCl and IBCF are particularly preferred.

A coupling additive is preferably a nucleophilic hydroxy compound capable of forming active esters. Coupling additives are known in the prior art and are for instance described in Houben-Weyl "Synthesis of Peptides and Peptidomimetics", Vol. E22a-E22e (Methods in Organic Chemistry), Thieme Publishing Group, 4th edition, 2001-2003. Preferred coupling additives are selected from the group consisting of *N-*hydroxysuccinimide (HOSu), *N-*hydroxy-5-norbornene-2,3-dicarboximide (HONb) and 1-hydroxybenzotriazole (HOBt). In particularly preferred embodiment HOSu is used in combination with the coupling reagent DCC, preferably in the peptide coupling steps a), b), aa), bb), dd) and ee).

The reaction between an amino acid or a peptide, a coupling reagent and a coupling additive leads to formation of an active ester. The reaction temperature for this transformation is chosen depending on the sensitivity of the amino acid or the peptide and of the resulting active ester. Thus, the reaction temperature is preferably chosen from -30°C to 30°C, more preferably from -20°C to 20°C, even more preferably from -10°C to 10°C. Under these conditions epimerisation of the stereogenic centre adjacent to the activated C-terminus and formation of undesired by-products such as *N-*acylureas are substantially suppressed. Once the coupling reagent has been added, the reaction temperature can be increased to a temperature between 5°C and 45°C, preferably between 10°C and 20°C.

The tertiary base employed in the peptide coupling steps a)-c) and aa)-gg) is not particularly limited and is preferably compatible with the products of the peptide coupling steps a)-c) and aa)-gg) and with the coupling reagent. Preferably, the conjugated acid of said tertiary base used in the peptide coupling reaction has a pKa value from 7.5 to 15, more preferably from 7.5 to 10. Said tertiary base is preferably selected from the group consisting of trialkylamines, such as *N,N-*diisopropylethylamine (DIPEA) or triethylamine (TEA) or *N-*C₁₋₄ alkylmorpholines, such as *N-*methylmorpholine, with any C₁₋₄ alkyl being identical or different and independently from each other unbranched or branched C₁₋₄ alkyl. DIPEA, TEA and *N-*methylmorpholine are particularly preferred as tertiary bases for the peptide coupling steps a)-c) and aa)-gg).

If a carbodiimide coupling reagent such as DCC or EDC is used as a coupling reagent, after completion of the peptide coupling step the excess of the coupling reagent is preferable decomposed by an addition of an acid, for instance acetic acid.

After completion of the peptide coupling step using the persilylation technique, the silylated compounds are preferable hydrolysed by an addition of a protic solvent such as water, methanol, acetic acid or a mixture thereof.

In a preferred embodiment after completion of the peptide coupling step *N,N-*dimethyl-1,3-propanediamine (DMAPA) is added to the reaction mixture in order to decompose unreacted active esters.

According to the present invention it has surprisingly been found that the products of the peptide coupling steps a)-c) and aa)-gg) are advantageously crystallized from solvents such as methyl-t-butyl ether (MTBE), diisopropyl ether (IPE), isopropanol and cyclohexane or from a combination thereof, whereby MTBE and isopropanol / cyclohexane mixture are particularly preferred. The resulting crystalline products have a purity of at least 90 % of surface area, preferably at least 95 % of surface area, more preferred at least 97 % of surface area and particularly preferred at least 98 % of surface area as determined by analytical HPLC.

In a preferred embodiment of the present invention at least one of the peptide coupling steps a)-c) and aa)-gg) employs persilylated amino acids or persilylated peptides as intermediates, whereby in the particularly preferred embodiment all peptide coupling steps a)-c) and aa)-gg) employ persilylated amino acids or persilylated peptides as intermediates. Hereinafter this embodiment is designated as "persilylation technique". Persilylated amino acids and persilylated peptides are obtained from the corresponding amino acids and peptides in the presence of a silylating agent. A persilylated amino acid or a persilylated peptide comprises at least one trialkylsilylamino substituent R₂R₃R₄SiNH- and can be described by the Formula (III) : R₂R₃R₄SiNH-Xaa8-OSiR₂R₃R₄ (III), wherein the substituents R₂, R₃ and R₄ can be alkyl, aryl, arylalkyl or alkenyl substituents having 1-12, preferably 1-7 carbon atoms and Xaa8 is an amino acid or a peptide.

Preferably, the substituents R₂, R₃ and R₄ are selected from the group of substituents consisting of methyl, ethyl, isopropyl, *tert*-butyl and phenyl. In a particularly preferred embodiment the substituents R₂, R₃ and R₄ are identical and are methyl or ethyl groups, preferably methyl groups. Examples of persilylated amino acids are *N*-trimethylsilyl glycine trimethylsilyl ester, L-*N-*trimethylsilyl alanine trimethylsilyl ester and L-*N-*trimethylsilyl phenylalanine trimethylsilyl ester.

Thus, a peptide coupling step employing a persilylated amino acid or a persilylated peptide as an intermediate comprises the following steps :
α) persilylation of an amino acid or a peptide H-Xaa8-OH to yield a persilylated amino acid or a persilylated peptide R₂R₃R₄Si-Xaa8-OSiR₂R₃R₄ ;
(β) activating of an amino acid or a peptide PG1-Xaa7-OH to form an active ester ;
γ) coupling of the active ester obtained in step β) with the persilylated amino acid or the persilylated peptide R₂R₃R₄Si-Xaa8-OSiR₂R₃R₄ obtained in step α) whereby the peptide PG1-Xaa7-Xaa8-OSiR₂R₃R₄ is formed.

The peptide PG1-Xaa7-XaaB-OSiR₂R₃R₄ is preferable hydrolysed by a subsequent addition of a protic solvent such as water, methanol, acetic acid or a mixture thereof.

The choice of a silylating agent used for the preparation of persilylated amino acids or persilylated peptides is not particularly limited as long as it is compatible with the reaction solvent and the PGs employed. The silylating agents are known in the prior art and are, for instance, described in the publication "Silylating Agents" by Fluka Chemie AG, Buchs, Switzerland, ISBN-3-905617-07-2, 1995. For instance, silylating agents such as *N*,*O*-bis(trimethylsilyl)acetamide, trimethylsilyl chloride, trimethylsilyl bromide, *N-*trimethylsilylacetamide (TMA), trimethylsilylcyanide (TMSCN) or hexamethyldisilazane can be used in the process of the present invention. If silylating agents such as trimethylsilyl bromide or trimethylsilyl chlorde are employed, step α) is preferably carried out in the presence of a tertiary base in order to neutralise the released acid. TMA is the particularly preferred silylating agent for the preparation of persilylated amino acids and persilylated peptides in step α).

Without wishing to be bound by any theory, it is believed that transforming an amino acid or a peptide to the corresponding persilylated amino acids or persilylated peptide improves the selectivity and the yield of the subsequent peptide coupling step γ). As a consequence, the resulting product can be isolated with a higher purity and in a better yield compared to the coupling of unsilylated amino acids or peptides. Moreover, when persilylated amino acids or persilylated peptides are used in the process of the present invention, a less extensive use of PGs is required. For instance, the side chains of tyrosine and arginine residues do not require any PGs in this embodiment. Furthermore, when persilylated amino acids or persilylated peptides are used, the steps involving protection and deprotection of the carboxylic acid groups of the obtained peptides prior to the subsequent peptide coupling steps are no longer necessary. This means that the synthesized peptide can be directly used for the next peptide coupling step. Consequently, the formation of undesired side-products resulting from amino acids and peptides with protected carboxylic acid groups is also avoided.

Moreover, persilylated amino acids and persilylated peptides usually have better solubility in common organic solvents than the corresponding amino acids and peptides. Consequently, the corresponding peptide coupling step does not necessarily require that a polar aprotic solvent such as DMF or DMA is used as a reaction solvent. The peptide coupling can be therefore carried out in a less polar reaction solvent such as MeCN, DCM, EtOAc, iPrOAc or mixtures thereof. Thus, the reaction solvent employed for the steps α)-γ) of the present invention is preferably selected from the group consisting of DCM, MeCN, DMA or mixtures thereof.

The reaction temperature for the preparation of persilylated amino acids or persilylated peptides is preferably chosen to be between 0°C and 100°C, more preferably between 10°C and 80°C and particularly preferred between 30°C and 70°C.

In one of preferred embodiments of the present invention the amino acid residues Xaa2 and Xaa5 are identical. In this preferred embodiment Xaa4 and Xaa6 are also identical. It is particularly preferred that Xaa2 and Xaa5 are glutamic acid residues and Xaa4 and Xaa6 are lysine residues.

Thus, in this particular embodiment the peptide moiety prepared by the process of the present invention comprises an amino acid sequence (IV)

H-Xaa1-Xaa2-Xaa3-Xaa4-Xaa1-Xaa2-Xaa3-Xaa4-R1 (IV)

wherein R1 is selected from OH and NH₂.

Accordingly, in this embodiment the process for preparing a peptide moiety comprising the amino acid sequence (IV) is particularly convergent and comprises the following peptide coupling steps aa)-dd) :
aa) coupling of PG1-Xaa1-OH and H-Xaa2-OH to form PG1-Xaa1-Xaa2-OH ;
bb1) coupling of PG1-Xaa3-OH and H-Xaa4-OH to form PG1-Xaa3-Xaa4-OH ;
bb2) coupling of PG1-Xaa3-OH and H-Xaa4-NH₂ to form PG1-Xaa3-Xaa4-NH₂ ;
cc) coupling of PG1-Xaa1-Xaa2-OH and H-Xaa3-Xaa4-OH or H-Xaa3-Xaa4-NH₂ to form PG1-Xaa1-Xaa2-Xaa3-Xaa4-OH or PG1-Xaa1-Xaa2-Xaa3-Xaa4-NH₂ ;
dd) coupling of PG1-Xaa1-Xaa2-Xaa3-Xaa4-OH and H-Xaa1-Xaa2-Xaa3-Xaa4-OH or H-Xaa1-Xaa2-Xaa3-Xaa4-NH₂ to form the peptide moiety comprising the amino acid sequence (IV).

In another preferred embodiment of the present invention the amino acid residues Xaa2 and Xaa4 are identical. In this preferred embodiment Xaa5 and Xaa6 are also identical. It is particularly preferred that Xaa2 and Xaa4 are glutamic acid residues and Xaa5 and Xaa6 are lysine residues.

Thus, in this embodiment the peptide moiety prepared by the process of the present invention comprises an amino acid sequence (V)

H-Xaa1-Xaa2-Xaa3-Xaa2-Xaa1-Xaa5-Xaa3-Xaa5-R1 (V)

wherein R1 is selected from OH or NH₂.

Accordingly, the process comprises the following peptide coupling steps aa)-gg) :
aa) coupling of PG1-Xaa1-OH and H-Xaa2-OH to form PG1-Xaa1-Xaa2-OH;
bb) coupling of PG1-Xaa3-OH and H-Xaa2-OH to form PG1-Xaa3-Xaa2-OH ;
cc) coupling of PG1-Xaa1-Xaa2-OH and H-Xaa3-Xaa2-OH to form PG1-Xaa1-Xaa2-Xaa3-Xaa2-OH;
dd) coupling of PG1-Xaa1-OH and H-Xaa5-OH to form PG1-Xaa1-Xaa5-OH ;
ee) coupling of PG1-Xaa3-OH and H-Xaa5-R1 to form PG1-Xaa3-Xaa5-R1 ;
ff) coupling of PG1-Xaa1-Xaa5-OH and H-Xaa3-Xaa5-R1 to form PG1-Xaa1-Xaa5-Xaa3-Xaa5-R1 ;
gg) coupling of PG1-Xaa1-Xaa2-Xaa3-Xaa2-OH and H-Xaa1-Xaa5-Xaa3-Xaa5-R1 to form the peptide moiety comprising the amino acid sequence (V).

In yet another preferred embodiment of the present invention the residues of amino acids Xaa1 and Xaa3 are identical and selected from the group consisting of valine, leucine, isoleucine, norleucine, phenylalanine, tryptophan and tyrosine residues.

Preferred peptide moieties comprise one type of hydrophobic amino acids and two types of charged amino acids. Especially suitable peptide moieties are formed by the combination of two sequences chosen independently from the group consisting of FEFE, FEFK, FEFD, FEFR, FRFR, FRFK, FRFE, FRFD, FKFE, FKFK, FKFR, FKFD, FDFD, FDFE, FDFR, FDFK, WEWE, WEWK, WRWR, WKWE, WEWR, WRWE, WKWR, WRWK, WDWD, WDWE, WEWD, WDWK, WKWD, WDWR, WRWD, IEIE, IEIK, IRIR, IKIE, IEIR, IRIE, IKIR, IRIK, IDID, IDFE, IEID, IDIK, IKID, IDIR, IRID, YEYE, YRYR, YEYK, YKYE, YEYR, YRYE, YKYR, YRYK, YDYD, YDYE, YEYD, YDYK, YKYD, YDYR, YRYD, Nle-E-Nle-E, Nle-K-Nle-K, Nle-R-Nle-R, Nle-E-Nle-K, Nle-K-Nle-E, Nle-E-Nle-R, Nle-R-Nle-E, Nle-K-Nle-R, Nle-R-Nle-K, Nle-D-Nle-D, Nle-D-Nle-E, Nle-E-Nle-D, Nle-D-Nle-K, Nle-K-Nle-D, Nle-D-Nle-R, and Nle-R-Nle-D. The two sequences can be the same or different, especially the same.

Thus the peptide moiety provided by the process of the present invention might for instance be selected from the group consisting of FEFKFEFK, FEFEFKFK, FDFKFDFK, FDFDFKFK, FEFRFEFR, FEFEFRFR, YDYKYDYK, YDYDYKYK, YEYRYEYR, YEYKYEYK, YEYEYKYK, WEWKWEWK, WEWEWKWK, WDWKWDWK, WDWDWKWK. Further, the peptide moiety provided by the process of the present invention is selected from the group consisting of FEFKFEFK-NH₂, FEFEFKFK-NH₂, FDFKFDFK-NH₂, FDFDFKFK-NH₂, FEFRFEFR-NH₂, FEFEFRFR-NH₂, YDYKYDYK-NH₂, YDYDYKYK-NH₂, YEYRYEYR-NH₂, YEYKYEYK-NH₂, YEYEYKYK-NH₂, WEWKWEWK-NH₂, WEWEWKWK-NH₂, WDWKWDWK-NH₂, WDWDWKWK-NH₂. The amino sequences FEFKFEFK, FEFKFEFK-NH₂, FEFEFKFK or FEFEFKFK-NH₂ are particularly preferred.

The peptide moiety prepared by the process of the present invention is preferably able to self-assemble into a secondary structure selected from the group consisting of a β-sheet, a coiled coil, an α-helix structure and a peptide triple helix structure or to a combination thereof, for instance into a β-sheet structure. Therefore, it can form a hydrogel when the corresponding peptide is provided in suitable conditions.

This feature of the peptide moiety is important for its use in biomaterials but it also renders handling and purification of the peptide moiety as well of some intermediates during its preparation challenging.

Since the peptides are brought into contact with water in the course of preparing the peptide, gel formation may cause problems. The authors of the present invention surprisingly found that the gel formation during the preparation of the peptide moiety can be efficiently avoided if the reaction solvent employed for the peptide coupling step contains between 5 vol.-% and 50 vol.-%, preferably between 10 vol.-% and 30 vol.-%, for example 20 vol.-%, of a polar aprotic solvent, such as DMA.

In summary, the process for preparing a peptide moiety provided by the present invention allows an efficient preparation of self-assembling peptides on an industrial scale. Moreover the self-assembling peptides obtained by this process have a higher purity in comparison to the self-assembling peptides, prepared by a SPPS.

Another aspect on the present invention relates to a process for synthesis of a polymer covalently linked with at least one peptide unit, wherein the peptide unit comprises the peptide moiety obtained by the process described above and a functional peptide moiety comprising a bioactive amino acid sequence.

The bioactive amino acid sequence comprises one or more sequences selected from the group consisting of RGD, Har-Gly-Asp, RGDS, GRGDS, GRGDY, GRGDF, YGRGD, GRGDSY, GRGDSP, GRGDSPK, YRGDS, GRGETP, GRGESP, GRGDTP, GRGDSP, GRGDK, GRADSPK, GGGGRGDS, GRGDNP, RGDYK, RGDFK, LDV, REDV, RGDV, LRGDN, IKVAV, YIGSR, PDSGR, RNAIEIIKDA, RGDT, DGEA, VTXG (with X equal to any amino acid except Pro), GHK, QHREDGS, RGDWP, Har-Gly-Asp-Trp-Pro, SDKP, (PPG)_{z}, (PEG)_{z}, (PDG)_{z}, (PKG)_{z}, (PRG)_{z} where z is 1-50 but not limited to it.

The process for synthesis of a polymer covalently linked with at least one peptide unit is carried out in liquid phase and comprises the following steps :
i) esterification of the *C*-terminal carboxylic acid group of the peptide moiety ;
ii) removal of the *N-*terminal protective group PG1 ;
iii) coupling of the compound obtained in step ii) with the functional peptide moiety, whereby said peptide unit is obtained ;
iv) covalent linking of the peptide unit obtained in step iii) with the polymer through a linkage selected from the group consisting of a thioether linkage, an amino linkage, an amido linkage, an ester linkage or an ether linkage.

Yet another aspect of the present invention relates to a process for the synthesis of the peptide moiety comprising an amino sequence (II) covalently linked with another self-assembling peptide moiety or with a functional peptide moiety, the process comprising the steps i)-iii) :
i) esterification of the *C*-terminal carboxylic acid group of the peptide moiety ;
ii) removal of the *N-*terminal protective group PG1 ;
iii) coupling of the compound obtained in step ii) with the functional peptide moiety or another self-assembling peptide moiety.

The reaction conditions chosen for the esterification of the C-terminal carboxylic acid group of the peptide moiety in step i) are not particularly limited and depend on the protective groups of the peptide moiety. For instance, when the amino acid side chains of the peptide moiety are protected with acid sensitive protective groups, such as Boc esterification of the C-terminal carboxylic acid group is preferably carried out under basic conditions. Thus, this step can be carried out in the presence of an alkylating agent such as iodomethane, iodoethane or dimethyl sulfate and an inorganic base such as for instance sodium carbonate, potassium carbonate or caesium carbonate. For example, step i) can be performed in the presence of iodoethane and caesium carbonate.

The conditions preferably employed for the removal of the *N-*terminal protective group PG1 depend on the properties of this protective group. In one preferred embodiment of the present invention PG1 is Z and therefore, step ii) is carried out under reducing conditions, preferably in the presence of a slight excess of PTSA. In yet another preferred embodiment PG1 is a Fmoc and step ii) is carried out in the presence of an organic base such as for instance piperidine.

The reaction conditions suitable for step iii) are not particularly limited and are known to a person skilled in the art. Preferably, carbodiimide coupling reagents such as DCC or EDC are employed and HOBt is used as preferred coupling additive.

Should the disclosure of any patents, patent applications, and publications which are incorporated herein by reference conflict with the description of the present application to the extent that it might render a term unclear, the present description shall take precedence.

### Description of the drawings

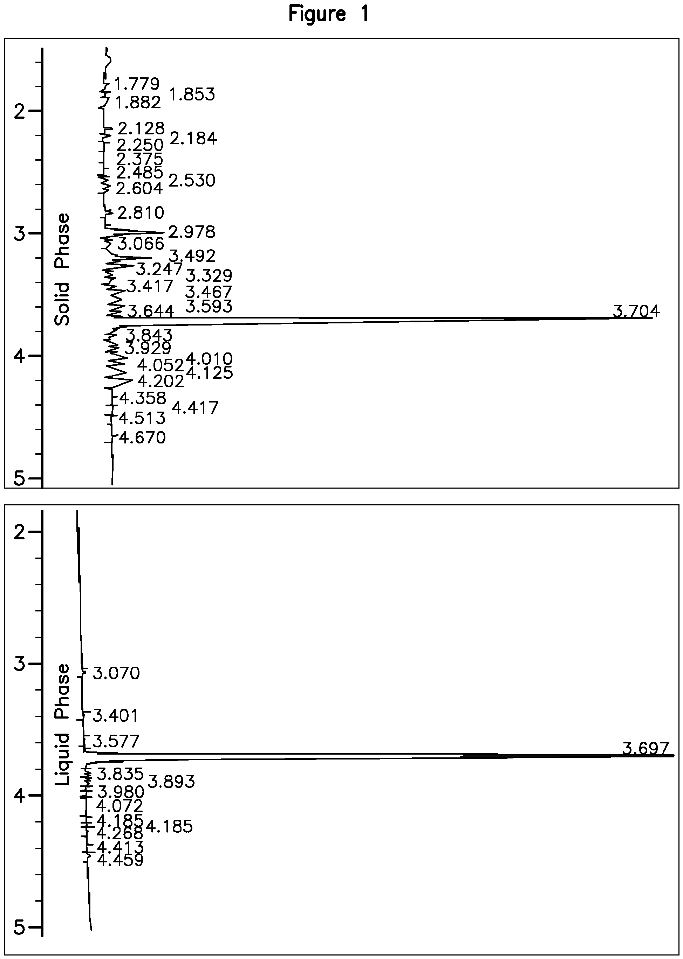

Figure 1 shows two HPLC chromatograms of the octapeptide Phe-Glu-Phe-Lys-Phe-Glu-Phe-Lys (FEFKFEFK). The sample corresponding to the chromatogram on the left hand side was synthesized by the protocol disclosed in A. Maslovskis et al. (Macromol. Symp., 296, pp. 248-253, 2010) (Comparative Example 2.3). The purity of this material was 63 % of surface area.

The sample, the chromatogram of which is shown on the right hand side was prepared according to the process of the present invention (Example 2.3). The purity of this material was 97% of surface area.

### Examples

All amino acids reagents as well as coupling reagents were used as purchased from commercial suppliers without further purification. If not specified otherwise, all operations were carried out at ambient temperature of 20±2°C and at atmospheric pressure of 101±5 kPa.

The water contents of the product samples were determined by volumetric Karl Fischer titration according to the standard test method ASTM E203-08.

### HPLC analysis

Eluent detection was performed with a UV photodiode array detector.
Mobile Phase A : water mQ, 0.1% TFA
Mobile Phase B : CH₃CN, 0.1% TFA

### Chromatography conditions :

Method CH-GS-8
Column : Merck Chromolith RP C18-e
Oven temperature : 40°C
Flow rate : 4.0 ml/min Detector wavelength : 220 nm
Gradient run time : 9.6 min
Gradient composition :

| **Time (min)** | **Solvent B (Vol.- %)** |
|---|---|
| 0.0 | 2.0 |
| 8.0 | 73.3 |
| 8.1 | 100.00 |
| 8.5 | 100.00 |
| 8.6 | 2.0 |
| 9.6 | 2.0 |

### Method CH-GS-10

Column : Merck Chromolith RP C18-e
Oven temperature : 40°C
Flow rate : 4.0 ml/min
Detector wavelength : 220 nm
Gradient run time : 9.6 min
Gradient composition :

| **Time (min)** | **Solvent B (Vol.- %)** |
|---|---|
| 0.0 | 2.0 |
| 10.0 | 91.6 |
| 10.1 | 100.00 |
| 10.5 | 100.00 |
| 10.6 | 2.0 |
| 11.6 | 2.0 |

### NMR analysis

The purity of the isolated product was determined by quantitative ¹H-NMR measurements in CD₃OD as a solvent. Octamethylcyclotetrasiloxane was used as an internal standard.

### 1. Synthesis of H-Phe-Glu-Phe-Lys-OH

### Example 1.1 Synthesis of Z-Phe-Glu(OtBu)-OH

521 g of Z-Phe-OH and 200 g of HOSu were dissolved in 5.71 of acetonitrile (CH₃CN). After cooling the solution to 0 ± 5°C, 359 g of *N,N'*-dicyclohexylcarbodiimide (DCC) were added thereto in small portions. At the end of the addition, the suspension was progressively brought back to ambient temperature (20°C). After HPLC control of the completion of the reaction, 5 g of acetic acid were added and the reaction mixture was further stirred for at least ½ h. After filtration of the *N,N'*-dicyclohexylurea (DCU), the solution of Z-Phe-OSu was added into a solution of 432 g of H-Glu(O*t*Bu)-OH previously silylated by 1123 g of *N-*trimethylsilyacetamide (TMA). 253 g of extra TMA are then poured into the solution. After checking the completion of the reaction by HPLC, 36 g of *N,N-*dimethylaminopropylamide (DMAPA) and 47 g of water were added to the reaction mixture. After a partial evaporation of CH₃CN and its replacement by EtOAc, the organic layer was successively washed with 5.21 of aqueous KHSO₄/NaCl, twice by 3.15 1 of water, and finally concentrated *under vacuum.* During the concentration, Z-Phe-Glu(O*t*Bu)-OH precipitated. The filtered solid was finally washed with 3.63 l of cyclohexane and dried at 45°C *under vacuum.* 549 g of Z-Phe-Glu(O*t*Bu)-OH as off-white solid were obtained.

The HPLC analysis of the product was carried out as follows : a sample was dissolved in 1 ml of methanol and analyzed by the method CH-GS-8 ; t_{R} = 5.6±0.5 min.

Product purity : 99 wt.- % (NMR), 100 % of surface area (HPLC).

Yield : 75 %.

### Example 1.2 Synthesis of Z-Phe-Lys(Boc)-OH

521 g of Z-Phe-OH and 200 g of HOSu were dissolved in 5.7 l of CH₃CN. After cooling the solution to 0 ± 5°C, 359 g of DCC were slowly added thereto. At the end of the addition, the suspension was progressively brought back to ambient temperature (20°C). After HPLC control of the reaction, 5 g of acetic acid were added and the reaction mixture was further stirred for at least ½ h. After filtration of the DCU, the solution of Z-Phe-OSu was added into a solution of 429 g of H-Lys(Boc)-OH previously silylated with 638 g of TMA. 357 g of extra TMA were then poured into the solution. After HPLC control of the reaction, 36 g of DMAPA and 47 g of water were added to the reaction mixture. After a partial evaporation of CH₃CN and its replacement by EtOAc, the organic layer was successively washed with 3.7 l of aqueous KHSO₄, twice with 6.1 l of water and finally concentrated *under vacuum.* After the concentration and cooling down, Z-Phe-Lys(Boc)-OH precipitated. The precipitate was diluted with 6 l diisopropylether (IPE). The filtered solid was finally washed with 3.63 l of IPE, and dried at 45°C *under vacuum.* 698 g of Z-Phe-Lys(Boc)-OH as white solid were obtained.

The HPLC analysis of the product was carried out as follows : a sample was dissolved in 1 ml of methanol and analyzed by the method CH-GS-8 ; t_{R} = 5.8±0.5 min.

Product purity : 97 wt.- % (NMR), 98 % of surface area (HPLC).

Yield : 74 %.

### Comparative Example 1.2 Preparation of Z-Phe-Lys(Boc)-OH using HOBt/DCC

An alternative synthetic protocol employing 1-hydroxybenzotriazole (HOBt) / DCC for the pre-activation of Z-Phe-OH was tested. After the pre-activation was complete, the silylated H-Lys(Boc)-OH was added thereto according to the procedure of Example 1.2. However, a substantial desilylation of the silylated H-Lys(Boc)-OH took place during the attempted coupling. This undesired reaction could not be suppressed by an addition of an excess of TMA to the reaction mixture. Moreover, a substantial formation of several by-products took place.

### Example 1.3 Synthesis of H-Phe-Lys(Boc)-OH

27 g of Pd/C (5 wt.- %) and 685 g of Z-Phe-Lys(Boc)-OH from Example 1.2 were dispersed in 537 ml of water and 7.3 l of methanol. The suspension was heated up to 50°C. After purging the reactor with nitrogen several times, hydrogen was introduced. After completion of the reaction as confirmed by HPLC, Pd/C was removed by filtration. The filtrated solution was then concentrated *under vacuum* and CH₃CN was progressively introduced during evaporation. The precipitated H-Phe-Lys(Boc)-OH was filtered, washed with 5.21 of CH₃CN and dried *under vacuum* at 45°C. 481 g of H-Phe-Lys(Boc)-OH were obtained as white solid.

The HPLC analysis of the product was carried out as follows : a sample was dissolved in 1 ml of methanol and analyzed by the method CH-GS-8 ; t_{R} = 3.4±0.5 min.

Product purity : 98 wt.- % (NMR), 95 % of surface area (HPLC).

Water content : 0.9 wt.- %.

Yield : 95 %.

### Example 1.4 Synthesis of Z-Phe-Glu(OtBu)-Phe-Lys(Boc)-OH

44.1 g of H-Phe-Lys(Boc)-OH obtained in Example 1.3 were added to a mixture of 68 g of TMA and 46 ml of dichloromethane (DCM). The suspension was heated to 50°C until solubilisation of H-Phe-Lys(Boc)-OH. 50 g of Z-Phe-Glu(O*t*Bu)-OH obtained in Example 1.1, 13.7 g of *N,N-*diisopropylethylamine (DIPEA), and 8.3 g of pyridine were solubilized in 649 ml of DCM and 32 ml of *N,N-*dimethylacetamide (DMA). After cooling down the solution to -10 ± 5°C, 12.7 g of pivaloyl chloride (PivCl) were added. After about 10 min, the cooled solution of silylated H-Phe-Lys(Boc)-OH was poured thereto. The reaction mixture was then progressively brought back to ambient temperature. After HPLC control, the organic layer was washed successively with 976 ml of 5 wt.- % aqueous KHSO₄, 972 ml of 5 wt.- % aqueous NaCl, and finally with 972 ml of water. The washed organic layer was concentrated *under vacuum,* and isopropanol was progressively introduced to it. The concentrated isopropanol phase was added drop wise to 1470 ml of cooled cyclohexane. The precipitate was filtered, washed with 143 ml of cyclohexane and dried at 45°C *under vacuum.* 67 g of Z-Phe-Glu(O*t*Bu)-Phe-Lys(Boc)-OH was obtained as an off-white product.

The HPLC analysis of the product was carried out as follows : a sample was dissolved in 1 ml of methanol and analyzed by the method CH-GS-10 ; t_{R} = 7.2±0.5 min.

Product purity : 99 wt.- % (NMR), 97 % of surface area (HPLC).

Yield : 78 %.

### Comparative Example 1.4 Isolation of Z-Phe-Glu(OtBu)-Phe-Lys(Boc)-OH

The preparation of Z-Phe-Glu(O*t*Bu)-Phe-Lys(Boc)-OH described above was accompanied by formation of by-product Piv-Phe-Lys(Boc)-OH (5 % of surface area by HPLC). Therefore, in order to isolate the desired product in a crystalline and sufficiently pure form, the crystallisation experiments in a number of solvents were carried out (s. Table 1 below).

**Table 1**

| **Solvent employed** | **Product appearance** |
|---|---|
| DCM | gel |
| EtOAc | gel |
| methanol | sticky oil |
| isopropanol | sticky oil |
| water | sticky resin |
| MTBE | gel |
| cyclohexane | gel |
| isopropanol / cyclohexane | crystalline material |

Surprisingly, the product could be isolated as a sufficiently pure crystalline material (97 % of surface area by HPLC) from a mixture of isopropanol and cyclohexane.

### Comparative Example 1.4-1 Preparation of Z-Phe-Glu(OtBu)-Phe-Lys(Boc)-OH

The synthetic protocol of Example 1.4 was modified in that the activation of Z-Phe-Glu(O*t*Bu)-OH was carried out in the presence of isobutyl chloroformiate and *N-*methyl morpholine. The isolated product yield was 64 % i.e. lower than in Example 1.4.

### Example 1.5 Synthesis of H-Phe-Glu(OtBu)-Phe-Lys(Boc)-OH

65 g of Z-Phe-Glu(O*t*Bu)-Phe-Lys(Boc)-OH from Example 1.4 were suspended in a mixture of 717 ml of methanol and 59 ml of water. By adding 13 g of KOH thereto, a solution was obtained. 1.6 g of Pd/C (5 wt.- %) were then dispersed in the solution. The suspension was heated up to 50°C. After purging the reactor with nitrogen several times, hydrogen was introduced. After completion of the reaction was verified by HPLC control, the Pd/C was removed by filtration. The filtrated solution was then partially concentrated *under vacuum.* The concentrated solution diluted with 443 ml of water was neutralized by adding 10.5 g of 37 wt.- % aqueous HCl. The precipitate was filtered, washed with 369 ml of water, and dried *under vacuum* at 45°C. 52 g of H-Phe-Glu(O*t*Bu)-Phe-Lys(Boc)-OH were obtained as a white solid.

The HPLC analysis of the product was carried out as follows : a sample was dissolved in 1 ml of methanol and analyzed by the method CH-GS-8 ; t_{R} = 5.0±0.5 min.

Product purity : 96 wt.- % (NMR), 96 % of surface area (HPLC).

Water content: 3 wt.- %.

Yield : 95 %.

### Example 1.6 Synthesis of H-Phe-Glu-Phe-Lys-OH

8.9 g of H-Phe-Glu(O*t*Bu)-Phe-Lys(Boc)-OH from Example 1.5 were poured into 89 ml of a TFA/water mixture (vol. ratio : 95/5). After at least 2 h, the deprotected peptide was precipitated in 300 ml methyl t-butyl ether (MTBE), washed with 100 ml MTBE and dried *under vacuum.*

### 2. Synthesis of H-Phe-Glu-Phe-Lys-Phe-Glu-Phe-Lys-OH

### Example 2.1 Synthesis of Z-[Phe-Glu(OtBu)-Phe-Lys(Boc)]₂-OH

41.6 g of H-Phe-Glu(O*t*Bu)-Phe-Lys(Boc)-OH obtained in Example 1.5 were added to a mixture of 65 g of TMA and 40 ml of DCM. The suspension was heated to 50°C until solubilisation of H-Phe-Glu(O*t*Bu)-Phe-Lys(Boc)-OH was complete. 43 g of Z-Phe-Glu(O*t*Bu)-Phe-Lys(Boc)-OH, 6.8 g of DIPEA and 4.1 g of pyridine were solubilized in 950 ml of DCM and 193 ml of DMA. After cooling down the solution to -10 ± 5°C, 6.0 g of PivCl were added. After 10 min, the cooled solution of silylated H-Phe-Glu(O*t*Bu)-Phe-Lys(Boc)-OH was poured thereto. The reaction mixture was then progressively brought back to ambient temperature (20°C). After HPLC control, the organic layer was added to 829 ml of 2.5 wt.- % aqueous KHSO₄. The suspension was partially concentrated *under vacuum.* The precipitate was filtered, washed with 731 ml of water, then with 432 ml of methanol, and dried at 45°C *under vacuum.* 62.7 g of Z-[Phe-Glu(O*t*Bu)-Phe-Lys(Boc)]₂-OH were obtained as an off-white product.

The HPLC analysis of the product was carried out as follows : a sample was dissolved in 1 ml of DMA and analyzed by the method CH-GS-10 ; t_{R} = 8.8±0.5 min.

Product purity : 96 wt.- % (NMR), 98 % of surface area (HPLC).

Yield : 80 %.

### Example 2.2 Synthesis of pTosOH·H-[Phe-Glu(OtBu)-Phe-Lys(Boc)]₂-OH

10 g of Z-[Phe-Glu(O*t*Bu)-Phe-Lys(Boc)]₂-OH from Example 2.1 and 0.14 g of *para*-toluenesulfonic acid (*p*TosOH) were introduced in 91.5 ml of DMA. 1.23 g of Pd/C (5 wt.- %) were then dispersed therein. The suspension was heated to 60°C. After purging the reactor with nitrogen several times, hydrogen was introduced. After the reaction completion was verified by HPLC control, Pd/C was removed by filtration. The filtrated solution was then added drop wise into 2000 ml of cooled IPE. The precipitate was filtered, washed with 500 ml of IPE, and dried *under vacuum* at 45°C. 10.7 g of *p*TosOH·H-[Phe-Glu(O*t*Bu)-Phe-Lys(Boc)]₂-OH were obtained as a white solid.

The HPLC analysis of the product was carried out as follows : a sample was dissolved in 1 ml of DMA and analyzed by the method CH-GS-10 ; t_{R} = 6.8±0.5 min.

Product purity : 72 wt.- % (NMR, 72 wt.- % peptide, 9.9 wt.- % PTSA, 7.4 wt.- % DMA and 2 wt.- % IPE), 97 % of surface area (HPLC).

Yield : 84 %.

### Example 2. 3 Synthesis of pTosOH·TFA·H-Phe-Glu-Phe-Lys-Phe-Glu-Phe-Lys-OH

10 g of *p*TosOH·H-[Phe-Glu(O*t*Bu)-Phe-Lys(BOC)]₂-OH obtained in Example 2.2 were dissolved in a mixture of 88 ml TFA and 4.6 ml water. After 2 h, the reaction mixture was partially evaporated *under vacuum* and poured into 1800 ml of cooled IPE. The precipitate was filtered, washed with 500 ml of IPE and dried *under vacuum* at 45°C. 9.3 g of *p*TosOH·TFA·H-Phe-Glu-Phe-Lys-Phe-Glu-Phe-Lys-OH were obtained as a white solid.

The HPLC analysis of the product was carried out as follows : a sample was dissolved in 1 ml DMA and analyzed by the method CH-GS-8 ; t_{R} = 3.7±0.5 min.

Product purity : 64 wt.- % (NMR, 64 wt.- % peptide, 9.8 wt.- % PTSA, 23.3 wt.- % TFA), 97 % of surface area (HPLC).

Yield : 98 %.

### Comparative Example 2.3 Preparation of H-Phe-Glu-Phe-Lys-Phe-Glu-Phe-Lys-OH in the solid phase

Octapeptide H-Phe-Glu-Phe-Lys-Phe-Glu-Phe-Lys-OH was synthesized as disclosed by A. Maslovskis et al. (Macromol. Symp., 296, pp.248-253, 2010), on a ChemTech ACT 90 peptide synthesizer using *N*-methyl-2-pyrrolidone (NMP) as solvent, and standard solid phase peptide protocols.

The obtained material was analysed by the same HPLC method as the material prepared in Example 2.3 and its purity was 63 % of surface area.

The chromatograms of both materials are shown in Figure 1. As can be recognized there from, the liquid phase synthesis of the present invention allows preparation of material having a significantly higher purity than the standard solid phase peptide protocol.

### Example 2.4 Esterification of the protected octapeptide

This esterification step was performed according to the procedure described in the literature : P. Jouin et al. (J. Org. Chem., 54, 3, pp. 617-627, 1989).

50 g of Z-Phe-Glu(OtBu)-Phe-Lys(Boc)-Phe-Glu(OtBu)-Phe-Lys(Boc)-OH (32 mmol) and 5.3 g of cesium carbonate (16 mmol) were introduced in 500 ml of DMF. 5.5 ml of iodoethane (69 mmol) were added, and the solution was heated at 48°C for 2 h. After filtration of the salts and partial evaporation of DMF, the concentrate was poured into 500 ml of 2.5 wt.- % aqueous KHSO₄, filtered, washed with water and finally with 500 ml warm ethanol. After drying under reduced pressure at 45°C, 47 g of a solid, corresponding to Z-Phe-Glu(OtBu)-Phe-Lys(Boc)-Phe-Glu(OtBu)-Phe-Lys(Boc)-OEt were obtained.

The HPLC analysis of the product was carried out as follows : a sample was dissolved in 1 ml DMA and analyzed by the method CH-GS-10 ; t_{R} = 9.0 ±0.5 min.

Product purity : 99 wt.- % (NMR), 97 % of surface area (HPLC).

Yield : 84 %.

### Example 2.5 Hydrogenolysis of the protected octapeptide ethyl ester

21.6 g of Z-Phe-Glu(OtBu)-Phe-Lys(Boc)-Phe-Glu(OtBu)-Phe-Lys(Boc)-OEt (13.6 mmol) obtained in Example 2.4 were dissolved in 215 ml of DMA. After flushing the solution several times with nitrogen, 14.5 g of Pd/Si (2 wt.- %) were added. Hydrogenolysis was initiated by the introduction of hydrogen. After 2 h of reaction, the suspension was passed through a 0.45 µm filter and Pd/Si was washed with DMA.

The combined filtrates, containing H-Phe-Glu(O*t*Bu)-Phe-Lys(Boc)-Phe-Glu(O*t*Bu)-Phe-Lys(Boc)-OEt can be used without further purification in the subsequent steps.

The yield was quantitative.

### 3. Synthesis of H-Phe-Glu-Phe-Lys-Phe-Glu-Phe-Lys-NH₂

### Example 3.1 Synthesis of Z-Phe-Lys(Boc)-NH₂

60 g of Z-Phe-OH and 23 g of HOSu were dissolved in 660 ml of CH₃CN. After cooling the solution to 0 ± 5°C, 41.3 g of DCC were added slowly thereto. At the end of the addition, the suspension was progressively brought back to ambient temperature. After the completion of the reaction was verified by HPLC control, 0.6 g of acetic acid were added and the reaction mixture was further stirred for at least ½ h. After filtration of the DCU, the solution of Z-Phe-OSu was added to a solution of 54 g of H-Lys(Boc)-NH₂ in 660 ml of CH₃CN. The immediate apparition of a precipitate was observed. After checking the completion of the reaction by HPLC, 5.05 ml of DMAPA were added to the reaction mixture. After a partial concentration of CH₃CN, the suspension was filtered, washed twice with 1000 ml of a mixture CH₃CN/water (vol. ratio : 80/20) and finally dried at 45°C *under vacuum.* 72.1 g of Z-Phe-Lys(Boc)-NH₂ were obtained as a white solid.

The HPLC analysis of the product was carried out as follows : a sample was dissolved in 1 ml DMA and analyzed by the method CH-GS-8 ; t_{R} = 6.0±0.5 min.

Product purity : 99 wt.- % (NMR), 100 % of surface area (HPLC).

Yield : 68 %.

### Example 3. 2 Synthesis of H-Phe-Lys(Boc)-NH₂

5.37 g of Z-Phe-Lys(Boc)-NH₂ from Example 3.1 were dissolved at 50°C in 58 ml of DMA. 1.1 g of Pd/C (5 wt.- %) were then dispersed in it. The suspension was heated up to 60°C. After purging the reactor with nitrogen several times, hydrogen was introduced. After the completion of reaction was verified by HPLC control, Pd/C was removed by filtration. This solution was used in the next step without further purification.

The yield was quantitative.

### Example 3.3 Synthesis of Z-Phe-Glu(OtBu)-Phe-Lys(Boc)-NH₂

4.75 g of Z-Phe-Glu(O*t*Bu)-OH from Example 1.1 were dispersed in the DMA solution of H-Phe-Lys(Boc)-NH₂ prepared in Example 3.2. 1.39 g of 1-hydroxybenzotriazole (HOBt) and 1.97 g of 1-ethyl-3-(3-dimethylaminopropyl) carbodiimide hydrochloride (EDC·HCl) were then added at 0°C. After the completion of the coupling was verified by HPLC control, the reaction mixture was precipitated in 835 ml of 5 wt.- % aqueous KHSO₄. The white precipitate was successively washed with 430 ml water, 400 ml of 5 wt.- % aqueous Na₂CO₃ and finally three times with 400 ml of water.

After drying *under vacuum* at 45°C, 7 g of Z-Phe-Glu(OtBu)-Phe-Lys(Boc)-NH₂ was obtained as a white powder.

The HPLC analysis of the product was carried out as follows : a sample was dissolved in 1 ml DMA and analyzed by the method CH-GS-8 ; t_{R} = 5.5±0.5 min.

Product purity : 99 wt.- % (NMR), 99 % of surface area (HPLC).

Yield : 83 %.

### Example 3.4 Synthesis of H-Phe-Glu(OtBu)-Phe-Lys(Boc)-NH₂

4 g of Z-Phe-Glu(O*t*Bu)-Phe-Lys(Boc)-NH₂ from Example 3.3 were dissolved at 50°C in 43 ml of DMA. 0.5 g of Pd/C (5 wt.- %) were then dispersed therein. The suspension was heated to 60°C. After purging the reactor with nitrogen several times, hydrogen was introduced. After the reaction completion was verified by HPLC control, Pd/C was removed by filtration. This solution was used in the next step without further purification.

The yield was quantitative.

### Example 3.5 Synthesis of Z-Phe-Glu(OtBu)-Phe-Lys(Boc)-Phe-Glu(OtBu)-Phe-Lys(Boc)-NH2

4.3 g of Z-Phe-Glu(O*t*Bu)-Phe-Lys(Boc)-OH from Example 1.4 was solubilized in the DMA solution of H-Phe-Glu(O*t*Bu)-Phe-Lys(Boc)-NH₂ obtained in Example 3.4. 667 mg of HOBt and 946 mg of EDC·HCl were then added. After the completion of the coupling was verified by HPLC control, the reaction mixture was precipitated in 1000 ml of 5 wt.- % aqueous KHSO₄. The white precipitate was successively washed with 400 ml water, 400 ml of 5 wt.- % aqueous NaHCO₃ and finally three times with 400 ml of water.

After drying *under vacuum* at 45°C, 6.3 g of Z-[Phe-Glu(O*t*Bu)-Phe-Lys(Boc)]₂-NH₂ was obtained as a white powder.

Yield : 81 %.

### Example 3. 6 Synthesis of pTosOH.H-[Phe-Glu(OtBu)-Phe-Lys(Boc)]₂-NH₂

4.15 g of Z-[Phe-Glu(O*t*Bu)-Phe-Lys(Boc)]₂-NH₂ and 0.5 g ofpTosOH were introduced in 55 ml of DMA. 2.7 g of Pd/C (5 wt.- %) were then dispersed. The suspension was heated to 60°C. After purging the reactor with nitrogen several times, hydrogen was introduced. After completion of the reaction was verified by HPLC control, Pd/C was removed by filtration. The filtrated solution was then poured into 350 ml of cooled IPE. The precipitate was filtered, washed with 75 ml of IPE, and dried *under vacuum* at 45°C. 3.26 g of *p*TosOH·H-[Phe-Glu(O*t*Bu)-Phe-Lys(Boc)]₂-NH₂ were obtained as an off-white solid.

Product purity : 86 wt.- % (NMR, 10.4 % pTos, 1.2 % DMA),

Yield : 75 %.

### Example 3.7 Synthesis of pTosOH·TFA·H-Phe-Glu-Phe-Lys-Phe-Glu-NH₂

3.26 g of *p*TosOH·H-[Phe-Glu(O*t*Bu)-Phe-Lys(Boc)]₂-NH₂ were dissolved in 31 ml TFA and 2 ml water. After 2 h of reaction, the reaction mixture was partially evaporated *under vacuum* and poured into 400 ml of cooled MTBE. The precipitate was filtered, washed with 100 ml of MTBE, and dried *under vacuum* at 45°C. 2.4 g of *p*TosOH·TFA·H-Phe-Glu-Phe-Lys-Phe-Glu-Phe-Lys-NH₂ were obtained as an off-white solid.

Yield : 85 %.

## Claims

1. A process for preparing a peptide moiety comprising an amino acid sequence (I)
H-Xaa1-Xaa5-Xaa3-Xaa6-R1 (I),
wherein R1 is selected from OH and NH₂ whereby Xaa1 and Xaa3 are residues of hydrophobic amino acids ; and
Xaa5 and Xaa6 are residues of charged amino acids,
whereby the process is carried out in liquid phase and comprises the following peptide coupling steps :
a) coupling of PG1-Xaa1-OH and H-Xaa5-OH to form PG1-Xaa1-Xaa5-OH;
b) coupling of PG1-Xaa3-OH and H-Xaa6-R1 to form PG1-Xaa3-Xaa6-R1 ; and
c) coupling of PG1-Xaa1-Xaa5-OH and H-Xaa3-Xaa6-R1 to form PG1-Xaa1-Xaa5-Xaa3-Xaa6-R1 ;
to form the peptide moiety comprising the amino acid sequence (I),
whereby PG1 is a *N*-terminal protective group.

2. A process for preparing a peptide moiety comprising an amino acid sequence (II)
H-Xaa1-Xaa2-Xaa3-Xaa4-Xaa1-Xaa5-Xaa3-Xaa6-R1 (II),
wherein R1 is selected from OH and NH₂
whereby Xaa1 and Xaa3 are residues of hydrophobic amino acids ; and
Xaa2, Xaa4, Xaa5 and Xaa6 are residues of charged amino acids,
whereby the process is carried out in liquid phase and comprises the following peptide coupling steps :
aa) coupling of PG1-Xaa1-OH and H-Xaa2-OH to form PG1-Xaa1-Xaa2-OH ;
bb) coupling of PG1-Xaa3-OH and H-Xaa4-OH to form PG1-Xaa3-Xaa4-OH ;
cc) coupling of PG1-Xaa1-Xaa2-OH and H-Xaa3-Xaa4-OH to form PG1-Xaa1-Xaa2-Xaa3-Xaa4-OH ;
dd) coupling of PG1-Xaa1-OH and H-Xaa5-OH to form PG1-Xaa1-Xaa5-OH ;
ee) coupling of PG1-Xaa3-OH and H-Xaa6-R1 to form PG1-Xaa3-Xaa6-R1 ;
ff) coupling of PG1-Xaa1-Xaa5-OH and H-Xaa3-Xaa6-R1 to form PG1-Xaa1-Xaa5-Xaa3-Xaa6-R1 ;
gg) coupling of PG1-Xaa1-Xaa2-Xaa3-Xaa4-OH and H-Xaa1-Xaa5-Xaa3-Xaa6-R1 to form the peptide moiety comprising the amino acid sequence (II),
whereby PG1 is a *N*-terminal protective group.

3. The process of claims 1 or 2, whereby Xaa1 and Xaa3 are selected from the group consisting of phenylalanine, tryptophan, histidine, tyrosine, leucine, isoleucine, norleucine, alanine and valine residues ; and
Xaa2, Xaa4, Xaa5 and Xaa6 are selected from the group consisting of arginine, aspartic acid, glutamic acid and lysine residues.

4. The process of any of claims 1 to 3, whereby the peptide coupling steps a)-c) and aa)-gg) employ persilylated amino acids or persilylated peptides as intermediates.

5. The process of any of claims 2 to 4, whereby the residues of amino acids Xaa2 and Xaa5 are identical and are glutamic acid residues ; and
Xaa4 and Xaa6 are identical and are lysine residues.

6. The process of any of claims 2 to 4, whereby the residues of amino acids Xaa2 and Xaa4 are identical and are glutamic acid residues ; and
Xaa5 and Xaa6 are identical and are lysine residues.

7. The process of any of claims 1 to 6, whereby the residues of amino acids Xaa1 and Xaa3 are identical and are selected from the group consisting of valine, leucine, isoleucine, norleucine, phenylalanine, tryptophan and tyrosine residues.

8. The process of any of claims 1 to 7, whereby the peptide moiety consists of 4 to 20 amino acid residues.

9. The process of any of claims 1 to 8, whereby the *N-*terminal protective group PG1 is cleavable under reducing conditions.

10. The process of any of claims 1 to 9, whereby the side chains of aspartic acid, glutamic acid and lysine residues are protected by protective groups cleavable under acidic conditions.

11. A process for synthesis of a polymer covalently linked with at least one peptide, wherein the peptide comprises the peptide moiety obtained by the process according to claims 1 to 10 and a functional peptide moiety comprising a bioactive amino acid sequence,
whereby the process is carried out in liquid phase and comprises the following steps :
i) esterification of the C-terminal carboxylic acid group of the peptide moiety;
ii) removal of the *N-*terminal protective group PG1 ;
iii) coupling of the compound obtained in step ii) with the functional peptide moiety, whereby said peptide is obtained ;
iv) covalent linking of the peptide obtained in step iii) with the thermoresponsive polymer through a linkage selected from the group consisting of a thioether linkage, an amino linkage, an amido linkage, an ester linkage or an ether linkage.
